# EUROPEAN PATENT APPLICATION

(11) **EP 2 404 591 A1**
(43) Date of publication of application: **11.01.2012**
(21) Application number: 10748486.7
(22) Date of filing: 02.03.2010
(51) Int. Cl.: A61K 8/37, A61K 8/34, A61K 8/55, A61Q 19/00, A61Q 19/10, C11D 7/26

(54) **COSMETIC PREPARATION, METHOD FOR PRODUCING SAME, COMPOSITION FOR COSMETIC PREPARATIONS, COSMETIC PREPARATION CONTAINING THE COMPOSITION FOR COSMETIC PREPARATIONS AND METHOD FOR PRODUCING SAME, AND CLEANSER FOR INDUSTRIAL USE**

(30) Priority: 03.03.2009 JP 2009049899
(71) Applicant: The Nisshin OilliO Group, Ltd., Tokyo 104-8285 (JP)
(72) Inventor: KOBAYASHI, Tatsuya, Yokohama-shi Kanagawa 235-8558 (JP); GOTO, Aki, Yokohama-shi Kanagawa 235-8558 (JP)
(74) Representative: Vuillermoz, Bruno
(86) International application number: PCT/JP2010/001387
(87) International publication number: WO 2010/100887

(57) **Abstract**

The present invention is related to: (1) a cosmetic preparation including an emulsifying agent, 1,3-propanediol (PDO), and a PDO difatty acid ester, wherein the fatty acids constituting the PDO difatty acid ester are one or two or more types of fatty acids selected from isostearic acid, n-octanoic acid, and n-decanoic acid, and a method for producing the cosmetic preparation; (2) a composition for cosmetic preparations, which includes the PDO difatty acid ester, a cosmetic preparation including the composition for cosmetic preparations, and a method for producing the cosmetic preparation; and (3) a cleanser for industrial use, which includes a PDO difatty acid ester that has fatty acid residues of n-octanoic acid and n-decanoic acid. The present invention is able to provide: a cosmetic preparation, a composition for cosmetic preparations, and a cosmetic preparation including the composition for cosmetic preparations, which have excellent cleansing properties, low irritation properties, moisturizing properties, and feeling of use, with reduced load on the environment; methods for producing the same; and, in addition, a cleanser for industrial use which has excellent cleansing properties and low corrosion properties, with reduced load on the environment.

## Description

### TECHNICAL FIELD

The present invention relates to: a cosmetic preparation including a dihydric alcohol and a difatty acid ester of a dihydric alcohol, and a method for producing the same; a composition for cosmetic preparations, which includes a difatty acid ester of an alcohol, a cosmetic preparation including the composition for cosmetic preparations, and methods for producing the same; and a cleanser for industrial use, which includes a difatty acid ester of an alcohol.
Priority is claimed on Japanese Patent Application No. 2009-049899, filed March 3, 2009, the content of which is incorporated herein by reference.

### BACKGROUND ART

In recent years, cosmetics are required to have makeup durability and excellent feeling of use. For this reason, many cosmetic preparations contain silicones and hydrocarbons that have a coating ability (hereunder, may be referred to as "silicones"). Cosmetics (makeups) using such cosmetic preparations can prevent the makeup from coming off as well as offering a feeling of use to fit the skin.
On the other hand, cleansing cosmetic preparations for use in the removal of such silicone-including cosmetics are required to have cleansing properties to remove these cosmetics without residue, low irritation properties to give little stimulation to the skin, moisturizing properties to prevent skin troubles, and a less sticky feeling of use (hereunder, may be referred to as "feeling of use").

So far, many cleansing cosmetic preparations contain anionic surfactants such as sodium lauryl sulfate (SLS). Because SLS has a quite high cleansing property, it is used not only for daily items such as toothpaste and shampoo, but is also often used for industrial purposes such as in the form of car wash detergent and engine oil remover. However, since SLS has a protein-denaturing effect, it is highly irritative to the skin. If SLS is contained a lot in a cleansing cosmetic preparation, the preparation can remove cosmetics but at the same time it also removes sebum that is necessary to the skin, and causes damages to the skin, which is a problem.
In addition, isononyl isononanoate, which is often contained in conventional types of cleansing cosmetic preparations, has relatively good cleansing properties, although the cleansing properties thereof are inferior to the cleansing properties of SLS. However, isononyl isononanoate is an ester that is often used as an emollient agent to soften the skin, and it is highly irritative to the skin, which is a problem.

Moreover, as an external preparation for the skin which is less irritative to the skin than SLS and isononyl isononanoate, tripropylene glycol esters of neopentanoic acid have been developed (refer to Patent Document 1). However, they are inferior when it comes to their cleansing properties, which is a problem.

In addition, cosmetic preparations are also required to have a small load on the environment in the production process, to follow the current worldwide trend. For example, it is considered that cosmetic preparations made of plant-derived materials or microbial fermentation-derived materials emit less carbon dioxide and thus have smaller loads on the environment in the production process, than those made of fossil fuel-derived materials. Moreover, it is considered that, if these cosmetic preparations are made of plant-derived materials or microbial fermentation-derived materials, the cosmetic preparations are easily decomposed by microorganisms and like matters residing in watercourses and soils when the preparations have been discharged to watercourses after use, and therefore the load on the environment is small.
Furthermore, differing from carbon contained in fossil fuels, carbon contained in industrial resources originated from components of living organisms such as plants and microorganisms is derived from carbon dioxide that has been absorbed from the atmosphere during the growth process of these living organisms through photosynthesis. For this reason, the use of such industrial resources is considered to produce no effect on the increase or decrease of the total amount of carbon dioxide in the atmosphere (hereunder, this concept may be referred to as the carbon neutral concept).
From the above-mentioned reasons, it is considered that, if these cosmetic preparations are made of plant-derived materials or microbial fermentation-derived materials, the load on the environment is small even when the cosmetic preparations have been discharged to the environment after use.

### CITATION LIST

### PATENT DOCUMENTS

Patent Document 1: WO 03/026698 pamphlet

### DISCLOSURE OF INVENTION

### PROBLEMS TO BE SOLVED BY THE INVENTION

The present invention was achieved by taking the above-mentioned situations into consideration with an object of providing: a cosmetic preparation which has excellent cleansing properties, low irritation properties, moisturizing properties, and feeling of use, with reduced load on the environment, and a method for producing the same; a composition for cosmetic preparations and a cosmetic preparation including the composition for cosmetic preparations, and methods for producing the same; and, in addition, a cleanser for industrial use which has excellent cleansing properties and low corrosion properties with reduced load on the environment.

### MEANS TO SOLVE THE PROBLEMS

The present invention relates to the following aspects, for example.
A first aspect of the present invention is a cosmetic preparation including an emulsifying agent, 1,3-propanediol, and a 1,3-propanediol difatty acid ester, wherein the fatty acids constituting the 1,3-propanediol difatty acid ester are one or two or more types of fatty acids selected from isostearic acid, n-octanoic acid, and n-decanoic acid.
A second aspect of the present invention is a method for producing the cosmetic preparation of the first aspect of the present invention, wherein the method comprises making an oil-in-water (O/W) emulsified cosmetic preparation by adding the 1,3-propanediol difatty acid ester to a hydrophilic solution in which the emulsifying agent, the 1,3-propanediol, and, if required, an auxiliary ingredient, have been previously dissolved or dispersed.
A third aspect of the present invention is a composition for cosmetic preparations, which includes a 1,3-propanediol difatty acid ester, wherein the fatty acids constituting the 1,3-propanediol difatty acid ester are one or two or more types of fatty acids selected from isostearic acid, n-octanoic acid, and n-decanoic acid.
A fourth aspect of the present invention is a cosmetic preparation including the composition for cosmetic preparations of the third aspect of the present invention.
A fifth aspect of the present invention is a method for producing the cosmetic preparation of the fourth aspect of the present invention, wherein the method comprises making an oil-in-water (O/W) emulsified cosmetic preparation by adding the composition for cosmetic preparations of the third aspect of the present invention, to a hydrophilic solution in which, if required, an auxiliary ingredient has been previously dissolved or dispersed.
A sixth aspect of the present invention is a cleanser for industrial use, which includes a 1,3-propanediol difatty acid ester, wherein the fatty acids constituting the 1,3-propanediol difatty acid ester are n-octanoic acid and n-decanoic acid.
A seventh aspect of the present invention is a method for producing the cosmetic preparation of the first aspect of the present invention, wherein the method comprises making a water-in-oil (W/O) emulsified cosmetic preparation by adding the 1,3-propanediol to a lipophilic solution in which the emulsifying agent, the 1,3-propanediol difatty acid ester, and, if required, an auxiliary ingredient, have been previously dissolved or dispersed.
An eighth aspect of the present invention is a method for producing the cosmetic preparation of the fourth aspect of the present invention, wherein the method comprises making a water-in-oil (W/O) emulsified cosmetic preparation by adding a hydrophilic solution in which, if required, an auxiliary ingredient has been previously dissolved or dispersed, to a lipophilic solution in which the composition for cosmetic preparations of the third aspect of the present invention has been previously dissolved or dispersed.

### EFFECT OF THE INVENTION

The cosmetic preparation, the composition for cosmetic preparations, and the cosmetic preparation including the composition for cosmetic preparations of the present invention have excellent cleansing properties, low irritation properties, moisturizing properties, and feeling of use, as well as being capable of reducing the load on the environment. Furthermore, with the method for producing a cosmetic preparation and the method for producing a cosmetic preparation including a composition for cosmetic preparations of the present invention, such cosmetic preparations as mentioned above and such cosmetic preparations including these kinds of compositions for cosmetic preparations as mentioned above can be obtained. In addition, the cleanser for industrial use of the present invention has excellent cleansing properties and low corrosion properties, as well as being capable of reducing the load on the environment.

### EMBODIMENTS FOR CARRYING OUT THE INVENTION

Hereunder is a detailed description of embodiments of the present invention.

The cosmetic preparation serving as the first aspect of the present invention includes an emulsifying agent, 1,3-propanediol, and a 1,3-propanediol difatty acid ester, and the fatty acids constituting the 1,3-propanediol difatty acid ester are one or two or more types of fatty acids selected from isostearic acid, n-octanoic acid, and n-decanoic acid.

The type of the cosmetic preparation of the first aspect of the present invention is not specifically limited as long as it is a cosmetic preparation required to have excellent cleansing properties, low irritation properties, moisturizing properties, or feeling of use, as its function. Preferred examples of this type of cosmetic preparation can be given by cleansing cosmetic preparations such as a cleansing gel, a cleansing cream, a face wash cream, and a shampoo; moisturizing cosmetic preparations such as a moisturizing gel, a moisturizing cream, a moisturizing emulsion, a skin lotion, a beauty essence, a shaving lotion, sun tanning/sun screening lotions, sun tanning/sun screening creams, a shaving cream, a hair rinse, a hair conditioner, a massage gel, and a moisturizing lip cream; and the like.

In the cosmetic preparation serving as the first aspect of the present invention, preferred aspects of the mass ratio of the 1,3-propanediol to the 1,3-propanediol difatty acid ester can be exemplified by 10:1 to 1:10, 3:1 to 1:8, 2:1 to 1:8, 2:1 to 1:7, 2:1 to 1:6, 1.8:1 to 1:6, and the like. If the ratio is within the above-mentioned range, the effect of the present invention can be improved.

If the cosmetic preparation of the first aspect of the present invention is a cleansing gel, a preferred aspect of the mass ratio of the 1,3-propanediol to the 1,3-propanediol difatty acid ester is from 10:1 1 to 3:2.

If the cosmetic preparation of the first aspect of the present invention is a moisturizing gel, a preferred aspect of the mass ratio of the 1,3-propanediol to the 1,3-propanediol difatty acid ester is from 3:1 to 1:8, another preferred aspect thereof is from 2:1 1 to 1:6, and yet another preferred aspect thereof is from 2:1 1 to 1:3.

If the cosmetic preparation of the first aspect of the present invention is a moisturizing emulsion, a preferred aspect of the mass ratio of the 1,3-propanediol to the 1,3-propanediol difatty acid ester is from 7:2 to 1:10, and another preferred aspect thereof is from 7:2 to 1:7.

If the cosmetic preparation of the first aspect of the present invention is a massage gel, a preferred aspect of the mass ratio of the 1,3-propanediol to the 1,3-propanediol difatty acid ester is from 3:1 to 1:8, and another preferred aspect thereof is from 3:2 to 1:5.

If the cosmetic preparation of the first aspect of the present invention is a moisturizing lip cream, a preferred aspect of the mass ratio of the 1,3-propanediol to the 1,3-propanediol difatty acid ester is from 3:1 to 1:20, another preferred aspect thereof is from 3:1 1 to 1:8, and yet another preferred aspect thereof is from 3: 1 to 1:6.

In the cosmetic preparation serving as the first aspect of the present invention, although the combination of two fatty acids constituting the 1,3-propanediol difatty acid ester is not specifically limited as long as the effect of the present invention is not impaired, combinations of "isostearic acid and isostearic acid" and "n-octanoic acid and n-decanoic acid" are preferred. If the 1,3-propanediol difatty acid ester has such a combination of these fatty acids, the effect of the present invention can be improved.
More specifically speaking, by including a 1,3-propanediol difatty acid ester having two isostearic acid residues (hereunder, may be abbreviated as PDO diisostearate) in the cosmetic preparation serving as the first aspect of the present invention, the moisturizing properties and the feeling of use of the cosmetic preparation can be more improved. In addition, by including a 1,3-propanediol difatty acid ester having an n-octanoic acid residue and an n-decanoic acid residue (hereunder, may be abbreviated as PDO-C8/C10 diester) in the cosmetic preparation serving as the first aspect of the present invention, the cleansing properties and the low irritation properties of the cosmetic preparation can be more improved.

Moreover, the cosmetic preparation serving as the first aspect of the present invention may also be a cosmetic preparation including both the PDO diisostearate and the PDO-C8/C10 diester. Such a cosmetic preparation including these two types of 1,3-propanediol difatty acid esters is able to have excellent moisturizing properties, feeling of use, cleansing properties, and low irritation properties.

The mass ratio of the n-octanoic acid and the n-decanoic acid which constitute the PDO-C8/C10 diester is preferably from 95:5 to 5:95, more preferably from 90:10 to 10:90, and yet more preferably from 80:20 to 20:80. If the ratio is within the above-mentioned range, the cleansing properties and the low irritation properties of the cosmetic preparation can be more improved.

A preferred aspect of the cosmetic preparation serving as the first aspect of the present invention can be given by a case where the fatty acids constituting the 1,3-propanediol difatty acid ester are n-octanoic acid and n-decanoic acid, and the 1,3-propanediol difatty acid ester is contained in a proportion of 5.0 to 60.0% by mass.

Another preferred aspect of the cosmetic preparation serving as the first aspect of the present invention can be given by a case where the fatty acids constituting the 1,3-propanediol difatty acid ester are n-octanoic acid and n-decanoic acid, the 1,3-propanediol difatty acid ester is contained in a proportion of 5.0 to 60.0% by mass, the emulsifying agent is contained in a proportion of 0.05 to 10.0% by mass, and the 1,3-propanediol is contained in a proportion of 3.0 to 50.0% by mass.

Yet another preferred aspect of the cosmetic preparation serving as the first aspect of the present invention can be given by a case where the fatty acids constituting the 1,3-propanediol difatty acid ester are isostearic acids, and the 1,3-propanediol difatty acid ester is contained in a proportion of 2.0 to 75.0% by mass.

Even yet another preferred aspect of the cosmetic preparation serving as the first aspect of the present invention can be given by a case where the fatty acids constituting the 1,3-propanediol difatty acid ester are isostearic acids, the 1,3-propanediol difatty acid ester is contained in a proportion of 2.0 to 75.0% by mass, the emulsifying agent is contained in a proportion of 0.05 to 10.0% by mass, and the 1,3-propanediol is contained in a proportion of 7.0 to 25.0% by mass.

If the cosmetic preparation serving as the first aspect of the present invention is, for example, a cleansing cosmetic preparation to remove an ink of an oil paint pen, a preferred aspect of the mass ratio of the n-octanoic acid and the n-decanoic acid which constitute the PDO-C8/C10 diester is from 90:10 to 40:60.

If the cosmetic preparation serving as the first aspect of the present invention is, for example, a cleansing cosmetic preparation to remove an ink of an oil paint pen, another preferred aspect of the mass ratio of the n-octanoic acid and the n-decanoic acid which constitute the PDO-C8/C10 diester is from 75:25 to 25:75.

It is preferable that the isostearic acid constituting the PDO diisostearate has a structure in which the principal chain thereof is bound with one or two methyl groups, in other words, one or two methyl groups are branched from the principal chain of stearic acid.
More specifically speaking, it is preferable that the isostearic acid is mainly such that any one of carbons from the second position to the sixteenth position of heptadecanoic acid is bound with a methyl group, or otherwise, any carbon(s) from the second position to the fifteenth position of hexadecanoic acid is(are) bound with two methyl groups, where these methyl groups may be bound to either the same carbon or different carbons. In addition, such heptadecanoic acid in which one methyl group is held in the principal chain, and such hexadecanoic acid in which two methyl groups are held in the principal chain, may each and singly constitute the PDO diisostearate, or may also be mixed to constitute the PDO diisostearate together. Furthermore, the isostearic acid may also be a by-product generated from the synthesis of a dimer acid from plant-derived oleic acid through a polymerization reaction performed by a usual method.
Moreover, the isostearic acid constituting the PDO diisostearate may also be obtained by a production method of isostearic acid, which includes: 1) a step of subjecting oleic acid to a polymerization reaction such as heat polymerization with use of montmorillonite white clay as a catalyst, if required; 2) a step of subjecting the yielded polymerization product to thin film distillation, so as to obtain a monomer acid; 3) a step of hydrogenating the monomer acid; and 4) a step of separating the thus hydrogenated monomer acid by a solvent fractionation method or the like, so as to obtain a product whose titer is 10°C or lower.
The PDO diisostearate can be obtained by esterifying the above-mentioned isostearic acid with 1,3-propanediol.
In the esterification reaction, the reaction temperature is usually from room temperature to 250°C, and preferably from 150 to 230°C. The reaction temperature is usually from 30 minutes to 24 hours, and preferably from 5 hours to 10 hours.
Furthermore, it is preferable that the isostearic acid constituting the PDO diisostearate has a viscosity of 99 to 130 mPa·s with a shear of 10 Pa at 10°C, and the isostearic acid is in a liquid state at 5°C. Examples of such isostearic acid satisfying these physical properties include "Isostearic Acid EX" manufactured by Kokyu Alcohol Kogyo Co., Ltd., "Prisorine 3501", "Prisorine 3505", and "Prisorine 3507", manufactured by Croda Inc., "Emersol 873", "Emersol 874", and "Emersol 875", manufactured by Cognis GmbH., "Radiacid 0907" and "Radiacid 0908" manufactured by Oleon Nv Company, and the like. It is either possible to use one of these products alone, or to use some of them as a mixture, as long as the physical properties are within the above-mentioned range.

If the isostearic acid has a structure in which one or two methyl groups are branched from the principal chain of stearic acid, the PDO diisostearate is liquid at room temperature (from 10 to 35°C). For this reason, it is possible to increase the proportion of the content of the PDO diisostearate while keeping the cosmetic preparation of the first aspect of the present invention in a liquid state. For example, it is possible to set the proportion of the PDO diisostearate in the cosmetic preparation to be about 60% by mass (however, this may not apply if other ingredient(s) differing from the PDO diisostearate included in the cosmetic preparation is(are) readily solidifiable).
Moreover, if the isostearic acid has a structure in which one or two methyl groups are branched from the principal chain of stearic acid, the moisturizing properties and the feeling of use of the cosmetic preparation can be more improved.
If the two isostearic acid residues held by the PDO diisostearate respectively have a structure in which one or two methyl groups are branched from the principal chain of stearic acid, the positions at which the methyl groups are bound may be either the same or different between these two isostearic acid residues.

In the cosmetic preparation serving as the first aspect of the present invention, the emulsifying agent can be exemplified by hydrogenated lecithins, trehalose isostearate esters, or the like. For example, hydrogenated lecithins are preferred for use in an oil-in-water (O/W) emulsified cosmetic preparation, while trehalose isostearate esters are preferred for use in a water-in-oil (W/O) emulsified cosmetic preparation.
The hydrogenated lecithins can be exemplified by a hydrogenated product of soybean-derived lecithin, a hydrogenated product of egg yolk-derived lecithin, or the like, although preferred is a hydrogenated product of soybean-derived lecithin from the viewpoint of the load on the environmental. The method to hydrogenate these lecithins is not specifically limited as long as the effect of the present invention is not impaired, and a known method is applied.
By using such a hydrogenated lecithin produced by hydrogenating soybean-derived lecithin, the 1,3-propanediol difatty acid ester can be sufficiently emulsified in the cosmetic preparation.
Such hydrogenated lecithins are preferred since their oxidative stability is higher than that of non-hydrogenated lecithins.
Moreover, such hydrogenated lecithins are preferred since their irritation property to the skin is relatively lower than that of synthetic surfactants that have been so far used as emulsifying agents of cosmetic preparations.

If the cosmetic preparation of the first aspect of the present invention is a cleansing cosmetic preparation, it is preferable that the 1,3-propanediol difatty acid ester included in the cosmetic preparation is a PDO diisostearate or a PDO-C8/C10 diester, and more preferably a PDO-C8/C10 diester. By using the PDO-C8/C10 diester as the 1,3-propanediol difatty acid ester, the cleansing properties and the low irritation properties of the cleansing cosmetic preparation can be more improved.

If the cosmetic preparation of the first aspect of the present invention is a cleansing cosmetic preparation, and if the 1,3-propanediol difatty acid ester is a PDO-C8/C10 diester; a preferred aspect of the proportions of the contents of maj or ingredients in the cosmetic preparation is such that the PDO-C8/C10 diester accounts for 40.0 to 60.0% by mass, the emulsifying agent accounts for 0.5 to 5.0% by mass, and the 1,3-propanediol accounts for 10.0 to 20.0% by mass; and another preferred aspect thereof is such that the PDO-C8/C10 diester accounts for 3.0 to 60.0% by mass, more preferably 3.0 to 30.0% by mass, and yet more preferably 5.0 to 30.0% by mass, the emulsifying agent accounts for 0.5 to 5.0% by mass, and the 1,3-propanediol accounts for 10.0 to 50.0% by mass, and more preferably 30.0 to 50.0% by mass.
By including the PDO-C8/C10 diester, the emulsifying agent, and the 1,3-propanediol, at such proportions within the above-mentioned range in the cleansing cosmetic preparation, then the effects of the cleansing properties and the low irritation properties of the cleansing cosmetic preparation can be even more improved. The cleansing cosmetic preparation having such proportions within the above-mentioned range is especially suitable for the application as a cleansing gel.

If the cosmetic preparation of the first aspect of the present invention is a cleansing cosmetic preparation, it is preferable to further include a polyglycerin fatty acid ester in the cosmetic preparation. By including the polyglycerin fatty acid ester, the cleansing properties of the cleansing cosmetic preparation can be yet even more improved.

The polyglycerin fatty acid ester is not specifically limited, as long as the effect of the present invention is not impaired, and as long as the polyglycerin fatty acid ester is able to improve the cleansing properties of the cleansing cosmetic preparation. Preferred are esterification products of one or two or more types of fatty acids selected from lauric acid, myristic acid, and oleic acid, with polyglycerin whose average degree of polymerization is 10, from the standpoint of improving the cleansing properties of the cleansing cosmetic preparation.
Of these, a preferred example of the polyglycerin fatty acid ester is an esterification product of lauric acid and polyglycerin whose average degree of polymerization is 10, with the saponification value of the esterification product being from 35 to 75, from the standpoint of improving the cleansing properties of the cleansing cosmetic preparation. In this case, the proportion of the content of the esterification product in the cleansing cosmetic preparation is preferably from 0.05 to 10.0% by mass, more preferably from 0.3 to 6.0% by mass, and most preferably from 0.5 to 3.0% by mass. If the proportion of the content is 0.05% by mass or higher, the cleansing properties of the cleansing cosmetic preparation can be improved. If the proportion of the content is 10.0% by mass or lower, the low irritation properties of the cleansing cosmetic preparation can be improved.

If the cosmetic preparation of the first aspect of the present invention is a moisturizing cosmetic preparation, it is preferable that the 1,3-propanediol difatty acid ester included in the cosmetic preparation is a PDO diisostearate or a PDO-C8/C10 diester, and more preferably a PDO diisostearate. By using the PDO diisostearate as the 1,3-propanediol difatty acid ester, the moisturizing properties and the feeling of use of the moisturizing cosmetic preparation can be more improved.

If the cosmetic preparation of the first aspect of the present invention is a moisturizing cosmetic preparation, and if the 1,3-propanediol difatty acid ester is a PDO diisostearate; a preferred aspect of the proportions of the contents of major ingredients in the cosmetic preparation is such that the PDO diisostearate accounts for 18.0 to 60.0% by mass, the emulsifying agent accounts for 0.5 to 5.0% by mass, and the 1,3-propanediol accounts for 10.0 to 20.0% by mass; and another preferred aspect thereof is such that the PDO diisostearate accounts for 3.0 to 75.0% by mass, more preferably 6.0 to 60.0% by mass, and yet more preferably 6.0 to 20.0% by mass, the emulsifying agent accounts for 0.5 to 5.0% by mass, and the 1,3-propanediol accounts for 10.0 to 20.0% by mass.
By including the PDO diisostearate, the emulsifying agent, and the 1,3-propanediol, at such proportions within the above-mentioned range in the moisturizing cosmetic preparation, then the moisturizing properties and the feeling of use of the moisturizing cosmetic preparation can be even more improved. The moisturizing cosmetic preparation having such proportions within the above-mentioned range is especially suitable for the application as a moisturizing gel.

If the cosmetic preparation of the first aspect of the present invention is a moisturizing cosmetic preparation, and if the 1,3-propanediol difatty acid ester is a PDO diisostearate; a preferred aspect of the proportions of the contents of major ingredients in the cosmetic preparation is such that the PDO diisostearate accounts for 5.0 to 18.0% by mass, the emulsifying agent accounts for 0.05 to 5.0% by mass, and the 1,3-propanediol accounts for 3.0 to 10.0% by mass; and another preferred aspect thereof is such that the PDO diisostearate accounts for 2.0 to 30.0% by mass, more preferably 2.0 to 18.0% by mass, the emulsifying agent accounts for 0.05 to 5.0% by mass, and the 1,3-propanediol accounts for 3.0 to 10.0% by mass.
By including the PDO diisostearate, the emulsifying agent, and the 1,3-propanediol, at such proportions within the above-mentioned range in the moisturizing cosmetic preparation, then the moisturizing properties and the feeling of use of the moisturizing cosmetic preparation can be even more improved. The moisturizing cosmetic preparation having such proportions within the above-mentioned range is especially suitable for the application as a moisturizing emulsion.

If the cosmetic preparation of the first aspect of the present invention is a moisturizing cosmetic preparation, and if the 1,3-propanediol difatty acid ester is a PDO-C8/C10 diester; a preferred aspect of the proportions of the contents of major ingredients in the cosmetic preparation is such that the PDO-C8/C10 diester accounts for 3.0 to 10.0% by mass, the emulsifying agent accounts for 0.05 to 5.0% by mass, and the 1,3-propanediol accounts for 3.0 to 10.0% by mass.
By including the PDO-C8/C10 diester, the emulsifying agent, and the 1,3-propanediol, at such proportions within the above-mentioned range in the moisturizing cosmetic preparation, it is possible to obtain a moisturizing cosmetic preparation whose feeling of use, low irritation properties, and/or storage stability are equivalent or better than those of cosmetic preparations in which a silicone oil is used as a base oil. The moisturizing cosmetic preparation having such proportions within the above-mentioned range is especially suitable for the application as a moisturizing emulsion.

If the cosmetic preparation of the first aspect of the present invention is a moisturizing cosmetic preparation, and if the 1,3-propanediol difatty acid ester is a PDO-C8/C10 diester; a preferred aspect of the proportions of the contents of major ingredients in the cosmetic preparation is such that the PDO-C8/C10 diester accounts for 20.0 to 50.0% by mass, the emulsifying agent accounts for 3.0 to 10.0% by mass, and the 1,3-propanediol accounts for 3.0 to 10.0% by mass.
By including the PDO-C8/C10 diester, the emulsifying agent, and the 1,3-propanediol, at such proportions within the above-mentioned range in the moisturizing cosmetic preparation, it is possible to obtain a moisturizing cosmetic preparation whose feeling of use, low irritation properties, and/or storage stability are equivalent or better than those of cosmetic preparations in which a silicone oil is used as a base oil. The moisturizing cosmetic preparation having such proportions within the above-mentioned range is especially suitable for the application as a moisturizing cream.
In addition, the above-mentioned moisturizing cosmetic preparation may also include 3.0 to 10.0% by mass of the PDO diisostearate, in addition to the PDO-C8/C10 diester.

If the cosmetic preparation of the first aspect of the present invention is a moisturizing cosmetic preparation, and if the 1,3-propanediol difatty acid ester is a PDO diisostearate; a preferred aspect of the proportions of the contents of major ingredients in the cosmetic preparation is such that the PDO diisostearate accounts for 5.0 to 60.0% by mass, and more preferably 8.0 to 50.0% by mass, the emulsifying agent accounts for 0.05 to 5.0% by mass, and the 1,3-propanediol accounts for 3.0 to 20.0% by mass.
By including the PDO diisostearate, the emulsifying agent, and the 1,3-propanediol, at such proportions within the above-mentioned range in the moisturizing cosmetic preparation, then the moisturizing properties and the feeling of use of the moisturizing cosmetic preparation can be even more improved. The moisturizing cosmetic preparation having such proportions within the above-mentioned range is especially suitable for the application as a massage gel.

If the cosmetic preparation of the first aspect of the present invention is a moisturizing cosmetic preparation, and if the 1,3-propanediol difatty acid ester is a PDO diisostearate; a preferred aspect of the proportions of the contents of major ingredients in the cosmetic preparation is such that the PDO diisostearate accounts for 5.0 to 70.0% by mass, and more preferably 8.0 to 60.0% by mass, the emulsifying agent accounts for 0.05 to 5.0% by mass, and the 1,3-propanediol accounts for 8.0 to 25.0% by mass.
By including the PDO diisostearate and the 1,3-propanediol at such proportions within the above-mentioned range in the moisturizing cosmetic preparation, then the moisturizing properties and the feeling of use of the moisturizing cosmetic preparation can be even more improved. The moisturizing cosmetic preparation having such proportions within the above-mentioned range is especially suitable for the application as a moisturizing lip balm.

The raw materials of the cosmetic preparation of the first aspect of the present invention are preferably derived from plant or microbial fermentation, and more preferably from plant. By not using a fossil fuel-derived material as a raw material of the cosmetic preparation, the load on the environment can be reduced. In addition, by using plant-derived materials as raw materials of the cosmetic preparation, the load on the environment can be much reduced.

The proportion of the plant-derived or microbial fermentation-derived raw materials in the entire raw materials of the cosmetic preparation of the first aspect of the present invention is preferably from 5 to 100% by mass, more preferably from 25 to 100% by mass, yet more preferably from 50 to 100% by mass, particularly preferably from 75 to 100% by mass, and most preferably 100% by mass (meaning that all the raw materials are substantially derived from plant or microbial fermentation).

The cosmetic preparation of the first aspect of the present invention may also include known ingredient(s) for use in a usual cosmetic preparation, for example, a moisturizing agent, a powder component, an ultraviolet absorber, an antioxidant, a beauty component, a glycolipid, a plant extract, an antiseptic agent, perfume, a pH regulator, a pigment, a viscosity modifier, a gelling agent, or the like, as auxiliary ingredient(s), within a range where the effect of the present invention is not impaired.

The moisturizing agent can be exemplified by propylene glycol, isoprene glycol, 1,2-pentanediol, 1,3-butylene glycol, dipropylene glycol, hexanediol, polyethylene glycol/glycerin, glycerin, diglycerin, triglycerin, polyglycerin, neopentyl glycol, sorbitol, erythritol, pentaerythritol, glucose, galactose, and such glycols, fructose, sucrose, maltose, xylose, xylobiose, a reduced oligosaccharide, a protein, a mucopolysaccharide, collagen, elastin, keratin, triethanolamine, and the like.

The powder component can be exemplified by inorganic white pigments such as titanium oxide, silicone-treated titanium oxide, zinc oxide, and barium sulphate; inorganic color pigments such as iron oxide, carbon black, sintered titanium/titanium oxide, and ultramarine; white powders such as talc, silicone-treated talc, muscovite mica, kaoline, silicon carbide, bentonite, smectite, anhydrous silica, aluminum oxide, magnesium oxide, zirconium oxide, diatomaceous earth, calcium silicate, barium silicate, magnesium silicate, calcium carbonate, magnesium carbonate, hydroxyapatite, and boron nitride; titanium dioxide-coated mica, iron oxide-coated titanated mica, silicone-treated titanated mica, argentine, powders of organic polymer resins such as a polyethylene based resin, a fluorine based resin, a cellulose based resin, and a silicone resin; low molecular organic powders such as zinc stearate and N-acyllysine; natural organic powders such as a starch, a silk powder, and a cellulose powder; organic pigment powders such as Red No. 201, Red No. 202, Orange No. 203, Orange No. 204, Blue No. 404, and Yellow No. 401; organic powder pigments of zirconium, barium, or aluminum lake, such as Red No. 3, Red No. 104, Red No. 106, Orange No. 205, Yellow No. 4, Yellow No. 5, Green No. 3, and Blue No. 1; metal powders such as mica and powdered gold; composite powders such as particulate titanium oxide-coated titanated mica; and the like.

The ultraviolet absorber can be exemplified by benzophenone derivatives, para-aminobenzoic acid derivatives, methoxycinnamic acid derivatives, urocanic acid, and the like.
The antioxidant can be exemplified by BHT, BHA, vitamins C, derivatives and salts thereof, vitamins E, derivatives and salts thereof, and the like.

The beauty component can be exemplified by vitamins including the above-mentioned types of vitamins, derivatives and salts thereof, antiphlogistic agents, galenicas, and the like.
The glycolipid can be exemplified by sphingoglycolipids and the like.
The plant extract can be exemplified by aloe vera, witch hazel, hamamelis, cucumber, lemon, lavender, rose, and the like.
The antiseptic agent can be exemplified by methyl parahydroxybenzoate, ethyl parahydroxybenzoate, butyl parahydroxybenzoate, propyl parahydroxybenzoate, phenoxyethanol, ethanol, and the like.

The perfume can be exemplified by a camphor oil, an orange oil, a peppermint oil, a jasmine absolute, a pine oil, a lime oil, a lavender oil, a rose oil, a musk tincture, and the like.
The pH regulator can be exemplified by edetic acid, disodium edetate, sodium chloride, citric acid, sodium citrate, sodium hydroxide, potassium hydroxide, triethanolamine, and the like.
The pigment can be exemplified by Blue No. 1, Blue No. 204, Red No. 3, Yellow No. 201, and the like.
The viscosity modifier can be exemplified by polyvinyl alcohol (PVA), methylcellulose (MC), ethylcellulose, hydroxypropyl methylcellulose, hydroxypropyl ethylcellulose, and other cellulose derivatives, polyvinylpyrrolidone (PVP), carboxymethyl cellulose, xanthan gum, alginic acid or salts thereof, carageenan, quince seed powder, *Alcaligenes*-produced polysaccharides, carboxyvinyl polymers, acrylic acid salts, acrylic acid polymers (chain type and crosslinked type), acrylic acid/methacrylic acid alkyl copolymers, and the like.
The gelling agent can be exemplified by glyceryl behenate/eicosadioate, polyglyceryl-10 behenate/eicosadioate, metal salts of fatty acids, hydroxystearic acid, dextrin fatty acid esters, inulin fatty acid esters, sucrose fatty acid esters, acylated cellobiose, dibenzylidene monosorbitol, amino acid-based gelling agents, silicic anhydride, organomodified clay minerals, fumed silica, alumina, crosslinked organopolysiloxane, hydrocarbon waxes such as a polyethylene wax and a paraffin wax, plant-based waxes such as a carnauba wax and a candelilla wax, agar, gelatin, and the like.

In the method for producing the cosmetic preparation of the first aspect of the present invention serving as the second aspect of the present invention, an oil-in-water (O/W) emulsified cosmetic preparation is made by adding the 1,3-propanediol difatty acid ester to a hydrophilic solution in which the emulsifying agent and the 1,3-propanediol have been previously dissolved or dispersed.

In the hydrophilic solution, if required, an auxiliary ingredient can be previously dissolved or dispersed. In this case, it is preferable to make the oil-in-water (O/W) emulsified cosmetic preparation by adding the 1,3-propanediol difatty acid ester to a hydrophilic solution in which the emulsifying agent, the 1,3-propanediol, and the auxiliary ingredient have been previously dissolved or dispersed, so as to produce the cosmetic preparation serving as the first aspect of the present invention.

The method to prepare the hydrophilic solution in which the emulsifying agent and the 1,3-propanediol have been previously dissolved or dispersed is not specifically limited as long as these respective ingredients are not damaged by the method. For example, it is possible to prepare the hydrophilic solution by adding these respective ingredients to purified water, and then subjecting the yielded solution to a heat treatment, an ultrasonic treatment, a stirring treatment using a disperser, and/or the like. Of these, a method using heat and stirring treatments is preferred since the respective ingredients can be efficiently dissolved or dispersed.
The proportion of the content of purified water in the hydrophilic solution is not specifically limited as long as the effect of the present invention is not impaired. Although the proportion may be 100% by mass, the proportion is preferably from 1 to 99.5% by mass, more preferably from 1 to 95% by mass, and yet more preferably from 1 to 90% by mass. If purified water is contained within the above-mentioned range, the auxiliary ingredient can be added to the hydrophilic solution as required.

In addition, the method to prepare the hydrophilic solution in which the emulsifying agent, the 1,3-propanediol, and the auxiliary ingredient have been previously dissolved or dispersed, is not specifically limited as long as these respective ingredients are not damaged by the method. For example, it is possible to prepare the hydrophilic solution by adding these respective ingredients to purified water, and then subjecting the yielded solution to a heat treatment, an ultrasonic treatment, a stirring treatment using a disperser, and/or the like. Of these, a method using heat and stirring treatments is preferred since the respective ingredients can be efficiently dissolved or dispersed.
Although the proportion of the content of purified water in the hydrophilic solution is not specifically limited as long as the effect of the present invention is not impaired, the proportion is preferably from 1 to 99.5% by mass, more preferably from 1 to 95% by mass, and yet more preferably from 1 to 90% by mass. If purified water is contained within the above-mentioned range, the cosmetic preparation can be efficiently made in the form of an oil-in-water (O/W) emulsified cosmetic preparation.

The auxiliary ingredient is not specifically limited as long as the effect of the present invention is not impaired. Preferred examples thereof can include the auxiliary ingredients, the emulsifying agents, and the 1,3-propanediol, which have been exemplified in the cosmetic preparation of the first aspect of the present invention, and the like.

The method to make such an oil-in-water (O/W) emulsified cosmetic preparation by adding the 1,3-propanediol difatty acid ester to the hydrophilic solution is not specifically limited, as long as the effect of the present invention is not impaired, and as long as the ingredients in the hydrophilic solution and the 1,3-propanediol difatty acid ester are not damaged. For example, the method is preferably such that the 1,3-propanediol difatty acid ester that has been heated to 60 to 80°C is gradually added to the hydrophilic solution that has been heated to 60 to 80°C under stirring, thereby making a mixed solution, and the mixed solution is cooled down to room temperature (from 20 to 25°C) under stirring so as to make the oil-in-water (O/W) emulsified cosmetic preparation.

Regarding the method of adding one or some of these auxiliary ingredients that is(are) preferably kept unheated, to the cosmetic preparation, preferred is a method in which the auxiliary ingredient(s) is(are) not previously dissolved or dispersed in the hydrophilic solution, but the auxiliary ingredient(s) is(are) added thereto at the point of time when the mixed solution has been cooled down to an appropriate temperature during the process of lowering the temperature of the mixed solution to room temperature.

The cosmetic preparation composition serving as the third aspect of the present invention is a composition for cosmetic preparations, which includes a 1,3-propanediol difatty acid ester, wherein the fatty acids constituting the 1,3-propanediol difatty acid ester are one or two or more types of fatty acids selected from isostearic acid, n-octanoic acid, and n-decanoic acid.

The type of the above-mentioned cosmetic preparation is not specifically limited as long as it is a cosmetic preparation required to have excellent cleansing properties, low irritation properties, moisturizing properties, or feeling of use, as its function. Preferred examples of this type of cosmetic preparation can be given by cleansing cosmetic preparations such as a cleansing gel, a cleansing cream, a face wash cream, and a shampoo; moisturizing cosmetic preparations such as a moisturizing gel, a moisturizing cream, a moisturizing emulsion, a skin lotion, a beauty essence, a shaving lotion, sun tanning/sun screening lotions, sun tanning/sun screening creams, a shaving cream, a hair rinse, a hair conditioner, a massage gel, and a moisturizing lip cream; and the like.

In the cosmetic preparation composition serving as the third aspect of the present invention, although the combination of two fatty acids constituting the 1,3-propanediol difatty acid ester is not specifically limited as long as the effect of the present invention is not impaired, combinations of "isostearic acid and isostearic acid" and "n-octanoic acid and n-decanoic acid" are preferred. If the 1,3-propanediol difatty acid ester has such a combination of these fatty acids, the effect of the present invention can be improved.
More specifically speaking, by including a 1,3-propanediol difatty acid ester having two isostearic acid residues (hereunder, may be abbreviated as PDO diisostearate) in the cosmetic preparation composition serving as the third aspect of the present invention, the moisturizing properties and the feeling of use of the cosmetic preparation composition can be more improved. In addition, by including a 1,3-propanediol difatty acid ester having an n-octanoic acid residue and an n-decanoic acid residue (hereunder, may be abbreviated as PDO-C8/C 10 diester) in the cosmetic preparation composition serving as the third aspect of the present invention, the cleansing properties and the low irritation properties of the cosmetic preparation composition can be more improved.

Moreover, the cosmetic preparation composition serving as the third aspect of the present invention may also be a cosmetic preparation composition which includes both the PDO diisostearate and the PDO-C8/C10 diester. Such a cosmetic preparation composition including these two types of 1,3-propanediol difatty acid esters is able to have excellent moisturizing properties, feeling of use, cleansing properties, and low irritation properties.

Preferred aspects of the mass ratio of the n-octanoic acid and the n-decanoic acid which constitute the PDO-C8/C10 diester can be exemplified by 95:5 to 5:95, 90:10 to 10:90, 80:20 to 20:80, and the like. If the ratio is within the above-mentioned range, the cleansing properties and the low irritation properties of the cosmetic preparation composition can be more improved.

If the cosmetic preparation composition serving as the third aspect of the present invention is, for example, a composition for cosmetic preparations to remove an ink of an oil paint pen, a preferred aspect of the mass ratio of the n-octanoic acid and the n-decanoic acid which constitute the PDO-C8/C10 diester is from 90:10 to 40:60.

It is preferable that the isostearic acid constituting the PDO diisostearate has a structure in which the principal chain thereof is bound with one or two methyl groups, in other words, one or two methyl groups are branched from the principal chain of stearic acid.
More specifically speaking, it is preferable that the isostearic acid is mainly such that any one of carbons from the second position to the sixteenth position of heptadecanoic acid is bound with a methyl group, or otherwise, any carbon(s) from the second position to the fifteenth position of hexadecanoic acid is(are) bound with two methyl groups, where these methyl groups may be bound to either the same carbon or different carbons. In addition, such heptadecanoic acid in which one methyl group is held in the principal chain, and such hexadecanoic acid in which two methyl groups are held in the principal chain, may each and singly constitute the PDO diisostearate, or may also be mixed to constitute the PDO diisostearate together. Furthermore, the isostearic acid may also be a by-product generated from the synthesis of a dimer acid from plant-derived oleic acid through a polymerization reaction performed by a usual method.
Moreover, the isostearic acid constituting the PDO diisostearate may also be obtained by a production method of isostearic acid, which includes: 1) a step of subjecting oleic acid to a polymerization reaction such as heat polymerization with use of montmorillonite white clay as a catalyst, if required; 2) a step of subjecting the yielded polymerization product to thin film distillation, so as to obtain a monomer acid; 3) a step of hydrogenating the monomer acid; and 4) a step of separating the thus hydrogenated monomer acid by a solvent fractionation method or the like, so as to obtain a product whose titer is 10°C or lower.
The PDO diisostearate can be obtained by esterifying the above-mentioned isostearic acid with 1,3-propanediol.
In the esterification reaction, the reaction temperature is usually from room temperature to 250°C, and preferably from 150 to 230°C. The reaction temperature is usually from 30 minutes to 24 hours, and preferably from 5 hours to 10 hours.
Furthermore, it is preferable that the isostearic acid constituting the PDO diisostearate has a viscosity of 99 to 130 mPa·s with a shear of 10 Pa at 10°C, and the isostearic acid is in a liquid state at 5°C. Examples of such isostearic acid satisfying these physical properties include "Isostearic Acid EX" manufactured by Kokyu Alcohol Kogyo Co., Ltd., "Prisorine 3501", "Prisorine 3505", and "Prisorine 3507", manufactured by Croda Inc., "Emersol 873", "Emersol 874", and "Emersol 875", manufactured by Cognis GmbH., "Radiacid 0907" and "Radiacid 0908" manufactured by Oleon Nv Company, and the like. It is either possible to use one of these products alone, or to use some of them as a mixture, as long as the physical properties are within the above-mentioned range.

If the isostearic acid has a structure in which one or two methyl groups are branched from the principal chain of stearic acid, the PDO diisostearate is liquid at room temperature (from 10 to 35°C). For this reason, it is possible to increase the proportion of the content of the PDO diisostearate while keeping the cosmetic preparation composition of the third aspect of the present invention in a liquid state. For example, it is possible to set the proportion of the PDO diisostearate in the cosmetic preparation composition to be about 60% by mass (however, this may not apply if other ingredient(s) differing from the PDO diisostearate included in the cosmetic preparation is(are) readily solidifiable).
Moreover, if the isostearic acid has a structure in which one or two methyl groups are branched from the principal chain of stearic acid, the moisturizing properties and the feeling of use of the cosmetic preparation composition can be more improved.
If the two isostearic acid residues held by the PDO diisostearate respectively have a structure in which one or two methyl groups are branched from the principal chain of stearic acid, the positions at which the methyl groups are bound may be either the same or different between these two isostearic acid residues.

The cosmetic preparation composition serving as the third aspect of the present invention may or may not include other ingredient(s) than the 1,3-propanediol difatty acid ester. In other words, it is either possible to use the cosmetic preparation composition serving as the third aspect of the present invention alone as a cosmetic preparation by itself, or to make a cosmetic preparation by having a combination of the cosmetic preparation composition and other ingredients(s).
The other ingredient(s) is(are) not specifically limited as long as the effect of the present invention is not impaired. Preferred examples thereof can include known ingredients for use in a usual cosmetic preparation, such as the auxiliary ingredients, the emulsifying agents, the 1,3-propanediol, and the like, which have been exemplified in the method for producing a cosmetic preparation of the second aspect of the present invention.
By including the cosmetic preparation composition serving as the third aspect of the present invention in a cosmetic preparation, the cleansing properties, the low irritation properties, the moisturizing properties, or the feeling of use of the cosmetic preparation can be improved.

If the cosmetic preparation composition serving as the third aspect of the present invention includes the PDO diisostearate, it is preferable to use the cosmetic preparation composition as a composition for cleansing cosmetic preparations, or a composition for moisturizing cosmetic preparations, and it is more preferable to use the cosmetic preparation composition as a composition for moisturizing cosmetic preparations.
By including the PDO diisostearate in such a cleansing cosmetic preparation or in such a moisturizing cosmetic preparation, the moisturizing properties and the feeling of use thereof can be improved.

If the cosmetic preparation composition serving as the third aspect of the present invention includes the PDO-C8/C10 diester, it is preferable to use the cosmetic preparation composition as a composition for cleansing cosmetic preparations, or as a composition for moisturizing cosmetic preparations, and it is more preferable to use the cosmetic preparation composition as a composition for cleansing cosmetic preparations.
By including the PDO-C8/C10 diester in such a cleansing cosmetic preparation or in such a moisturizing cosmetic preparation, the cleansing properties and the low irritation properties thereof can be improved.

Moreover, if the cosmetic preparation composition serving as the third aspect of the present invention includes the PDO diisostearate, it is also preferable to use the cosmetic preparation composition as a substitute for a cosmetic preparation composition including a macadamia nut oil.

The macadamia nut oil is a pale yellow oil in a liquid form, which is yielded from the nut (seed) of the macadamia, and contains oleic acid and palmitoleic acid that are unsaturated fatty acids as major ingredients.
Since the above-mentioned cosmetic preparation composition including a macadamia nut oil excels in the moisturizing properties and the feeling of use when applied to the skin, it is often used as a composition for moisturizing cosmetic preparations such as a massage oil, a beauty oil, a baby oil, or a cream, and as a composition for cleansing cosmetic preparations such as a cleansing gel or a soap.
However, a problem is that unsaturated fatty acids serving as the major ingredients of the macadamia nut oil are inferior in the oxidative stability. A cosmetic preparation composition including an oxidized macadamia nut oil is inferior in the moisturizing properties and the feeling of use.

The PDO diisostearate has quite similar viscosity and dynamic friction coefficient to those of the macadamia nut oil. For this reason, the moisturizing properties and the feeling of use in a case where a cosmetic preparation composition including the PDO diisostearate is applied to the skin are equivalent to those of a case where a cosmetic preparation composition including the macadamia nut oil is applied to the skin.
In addition, since the isostearic acids serving as the fatty acids constituting the PDO diisostearate are saturated fatty acids, the oxidative stability is excellent.
Accordingly, the cosmetic preparation composition including the PDO diisostearate serving as the cosmetic preparation composition of the third aspect of the present invention is useful as a substitute for a cosmetic preparation composition including the macadamia nut oil.

Moreover, if a cosmetic preparation composition serving as the third aspect of the present invention includes the PDO-C8/C10 diester, it is also preferable to use the cosmetic preparation composition to remove an oil soluble colorant. In other words, it is also preferable to include a cosmetic preparation composition which includes the PDO-C8/C10 diester, in a cosmetic preparation to be used for the purpose of removing an oil soluble colorant. By including such a cosmetic preparation composition which includes the PDO-C8/C10 diester, in the cosmetic preparation, the cleansing properties and the low irritation properties thereof can be improved.

The oil soluble colorant can be exemplified by cosmetics such as eye liner, mascara, eye shadow, eyebrow pencil, lipstick, lip gloss, lip balm, foundation cream, cheek color, manicure, and nail enamel; writing utensils such as oil marker pens, oil ballpoint pens, oil fountain pens, oil colors, oil paints, and oil crayons; and the like.

By using the cosmetic preparation composition including the PDO-C8/C10 diester of the third aspect of the present invention as an ingredient to remove an oil soluble colorant in a cosmetic preparation, it is possible to efficiently wash and remove the applied oil soluble colorant without damaging the skin surface to which the oil soluble colorant has been applied.
In other words, if the cosmetic preparation composition including the PDO-C8/C10 diester of the third aspect of the present invention is used in a cosmetic preparation for the purpose of removing an oil soluble colorant, the resulting product surpasses conventional types of cosmetic preparation compositions in the cleansing properties (property to remove the oil soluble colorant) and the low irritation properties (property of not damaging the surface applied with the oil soluble colorant).

If the cosmetic preparation composition including the PDO-C8/C10 diester is used as such an ingredient to remove an oil soluble colorant, the proportion of the content of the PDO-C8/C10 diester in the cosmetic preparation composition is preferably from 20 to 100% by mass, more preferably from 50 to 100% by mass, yet more preferably from 80 to 100% by mass, and may also be 100% by mass.
By having the proportion of the content within the above-mentioned range, the cleansing properties and the low irritation properties of the agent to remove an oil soluble colorant, which includes the cosmetic preparation composition, can be more improved.

Furthermore, if the cosmetic preparation composition serving as the third aspect of the present invention includes the PDO-C8/C10 diester, it is also preferable to use the cosmetic preparation composition to remove an ink of an oil paint pen. In other words, it is also preferable to include the PDO-C8/C10 diester in a cosmetic preparation to be used for the purpose of removing an ink of an oil paint pen. By including the PDO-C8/C 10 diester in the cosmetic preparation, the cleansing properties and the low irritation properties thereof can be improved.

For example, it is difficult for a conventional type of cosmetic preparation, a conventional type of oil agent, or the like, to erase letters, paintings, or marks, painted on the skin with an ink of a usual oil paint pen. This is because, if a conventional type of cosmetic preparation, oil agent, or the like having a high ink remover property is used, the ink could be removed whereas the skin surface painted with the oil paint pen might be damaged.

However, if the cosmetic preparation composition including the PDO-C8/C10 diester of the third aspect of the present invention, is used as such an ingredient to remove an ink of an oil paint pen in a cosmetic preparation, it is possible to efficiently remove the ink of the oil paint pen without damaging the skin surface that has been painted with the oil paint pen.
In other words, if the cosmetic preparation composition including the PDO-C8/C10 diester of the third aspect of the present invention is used as a cosmetic preparation for the purpose of removing an ink of an oil paint pen, the resulting product surpasses conventional types of cosmetic preparation compositions in the cleansing properties (property to remove the ink) and the low irritation properties (property of not damaging the surface painted with the oil paint pen).

If the cosmetic preparation composition including the PDO-C8/C10 diester is used as such an ingredient to remove an ink of an oil paint pen, the proportion of the content of the PDO-C8/C10 diester in the cosmetic preparation composition is preferably from 20 to 100% by mass, more preferably from 50 to 100% by mass, yet more preferably from 80 to 100% by mass, and may also be 100% by mass.
By having the proportion of the content within the above-mentioned range, the cleansing properties and the low irritation properties of the agent to remove an ink of an oil paint pen, which includes the cosmetic preparation composition, can be more improved.

Furthermore, if the cosmetic preparation composition serving as the third aspect of the present invention includes the PDO-C8/C10 diester, it is also preferable to use the cosmetic preparation composition as a substitute for a silicone oil, and more preferably a substitute for a low viscosity silicone oil. In other words, it is preferable that the PDO-C8/C10 diester is included in a cosmetic preparation to be used for the purpose of substituting for a silicone oil, such as a low viscosity silicone oil, which is used as a base oil of many conventional types of cosmetic preparations. Since (low viscosity) silicone oils are highly safe for the skin, they are used as base oils of many types of cosmetic preparations. However, silicone oils are originated from minerals, and, in addition, the recalcitrance of such silicone oils would impose large loads on the environment when cosmetic preparations using them are emitted to the environment. By including the PDO-C8/C10 diester instead of a silicone oil in a cosmetic preparation, it is possible to have equivalent or better feeling of use, low irritation properties, and/or storage stability of the cosmetic preparation than those of cosmetic preparations using a silicone oil.

The raw materials of the cosmetic preparation composition serving as the third aspect of the present invention are preferably derived from plant or microbial fermentation, and more preferably from plant. By not using a fossil fuel-derived material as a raw material of the cosmetic preparation composition, the load on the environment can be reduced. In addition, by using plant-derived materials as raw materials of the cosmetic preparation composition, the load on the environment can be much reduced.

The proportion of the plant-derived or microbial fermentation-derived raw materials in the entire raw materials of the cosmetic preparation composition serving as the third aspect of the present invention is preferably from 5 to 100% by mass, more preferably from 25 to 100% by mass, yet more preferably from 50 to 100% by mass, particularly preferably from 75 to 100% by mass, and most preferably 100% by mass (meaning that all the raw materials are substantially derived from plant or microbial fermentation).

The cosmetic preparation serving as the fourth aspect of the present invention includes the cosmetic preparation composition of the third aspect of the present invention.
The cosmetic preparation serving as the fourth aspect of the present invention is not specifically limited as long as it includes the cosmetic preparation composition of the third aspect of the present invention. Preferred examples thereof can include the cleansing cosmetic preparations and the moisturizing cosmetic preparations mentioned above, and, in addition, other preferred examples thereof can also include the cosmetic preparation to be used for the purpose of removing an oil soluble colorant and the cosmetic preparation to be used for the purpose of removing an ink of an oil paint pen mentioned above.
Moreover, it is preferable that the cosmetic preparation serving as the fourth aspect of the present invention is similar to the cosmetic preparation of the first aspect of the present invention.

In the method for producing a cosmetic preparation serving as the fifth aspect of the present invention, an oil-in-water (O/W) emulsified cosmetic preparation is made by adding the cosmetic preparation composition of the third aspect of the present invention, to a hydrophilic solution.

The hydrophilic solution is not specifically limited as long as the effect of the present invention is not impaired. A preferred example thereof can be purified water.
Moreover, in the hydrophilic solution, if required, an auxiliary ingredient can be previously dissolved or dispersed. In this case, it is preferable to make the oil-in-water (O/W) emulsified cosmetic preparation by adding the cosmetic preparation composition of the third aspect of the present invention, to a hydrophilic solution in which the auxiliary ingredient has been previously dissolved or dispersed, so as to produce the cosmetic preparation serving as the fifth aspect of the present invention.

The method to prepare the hydrophilic solution in which the auxiliary ingredient has been previously dissolved or dispersed is not specifically limited as long as the auxiliary ingredient is not damaged by the method. For example, it is possible to prepare the solution by adding the auxiliary ingredient to purified water, and then subjecting the yielded solution to a heat treatment, an ultrasonic treatment, a stirring treatment using a disperser, and/or the like. Of these, a method using a heat treatment is preferred since the auxiliary ingredient can be efficiently dissolved or dispersed.
The proportion of the content of purified water in the hydrophilic solution is not specifically limited as long as the effect of the present invention is not impaired, although the proportion is preferably from 1 to 99.5% by mass, more preferably from 1 to 95% by mass, and yet more preferably from 1 to 90% by mass. If purified water is contained within the above-mentioned range, the auxiliary ingredient can be added to the hydrophilic solution as required.

The auxiliary ingredient is not specifically limited as long as the effect of the present invention is not impaired. Preferred examples thereof can include the auxiliary ingredients, the emulsifying agents, and the 1,3-propanediol, which have been exemplified in the cosmetic preparation of the first aspect of the present invention, and the like.

The method to make such an oil-in-water (O/W) emulsified cosmetic preparation by adding the cosmetic preparation composition of the third aspect of the present invention to the hydrophilic solution is not specifically limited, as long as the effect of the present invention is not impaired, and as long as the ingredients in the hydrophilic solution and the cosmetic preparation composition of the third aspect of the present invention are not damaged. For example, the method is preferably such that the cosmetic preparation composition of the third aspect of the present invention that has been heated to 60 to 80°C is gradually added to the hydrophilic solution that has been heated to 60 to 80°C under stirring, thereby making a mixed solution, and the mixed solution is cooled down to room temperature (from 20 to 25°C) under stirring so as to make the oil-in-water (O/W) emulsified cosmetic preparation.

Regarding the method of adding one or some of these auxiliary ingredients that is(are) preferably kept unheated, to the cosmetic preparation, preferred is a method in which the auxiliary ingredient(s) is(are) not previously dissolved or dispersed in the solution, but the auxiliary ingredient(s) is(are) added thereto at the point of time when the mixed solution has been cooled down to an appropriate temperature during the process of lowering the temperature of the mixed solution to room temperature.

The industrial use cleanser serving as the sixth aspect of the present invention is a cleanser for industrial use, which includes a 1,3-propanediol difatty acid ester, wherein the fatty acids constituting the 1,3-propanediol difatty acid ester are n-octanoic acid and n-decanoic acid.

In the industrial use cleanser serving as the sixth aspect of the present invention, the combination of two fatty acids constituting the 1,3-propanediol difatty acid ester is a combination of "n-octanoic acid and n-decanoic acid".
By including a 1,3-propanediol difatty acid ester having an n-octanoic acid residue and an n-decanoic acid residue (hereunder, may be abbreviated as PDO-C8/C10) in the industrial use cleanser, the cleansing properties and the low corrosion properties of the industrial use cleanser can be more improved.

The mass ratio of the n-octanoic acid and the n-decanoic acid which constitute the PDO-C8/C10 diester is preferably from 95:5 to 5:95, more preferably from 90:10 to 10:90, and yet more preferably from 80:20 to 20:80. If the ratio is within the above-mentioned range, the cleansing properties and the low corrosion properties of the industrial use cleanser can be even more improved.

If the industrial use cleanser serving as the sixth aspect of the present invention is, for example, an industrial use cleanser to remove an ink of an oil paint pen, a preferred aspect of the mass ratio of the n-octanoic acid and the n-decanoic acid which constitute the PDO-C8/C10 diester is from 90:10 to 40:60.

The industrial use cleanser serving as the sixth aspect of the present invention may also include other ingredient(s) than the 1,3-propanediol difatty acid ester.
The other ingredient(s) is(are) not specifically limited as long as the effect of the present invention is not impaired. Preferred are known ingredients for use in a usual industrial use cleanser, such as an anionic surfactant, a cationic surfactant, a nonionic surfactant, an amphoteric surfactant, an oil agent, an alkali agent, a powder component, a viscosity modifier, a gelling agent, a sequestering agent, an antiseptic agent, a perfume, a pH regulator, a pigment, and the like.

The anionic surfactant can be exemplified by sodium alkylbenzene sulfonates, α-olefin sulfonic acid salts, alkali salts of higher fatty acids, alkyl sulfuric acid salts, alkyl ether sulfuric acid ester salts and ethylene oxide (EO) adducts thereof, alkyl ether phosphoric acid ester salts and ethylene oxide (EO) adducts thereof, phenyl ether sulfuric acid ester salts, methyltaurine acid salts, alaninates and salts thereof, sulfosuccinic acid salts, ether sulfuric acid salts, ether carboxylic acids and salts thereof, alkyl sulfonic acids and salts thereof, alkylbenzene sulfonic acids and salts thereof, and the like.

The cationic surfactant can be exemplified by quaternary ammonium salts typified by alkyl trimethyl ammonium chlorides and dialkyl dimethyl ammonium chlorides, and ethylene oxide (EO) adducts thereof; amine salts represented by the formula of [R-NH₃]⁺[CH₃COO]⁻ or the like, and ethylene oxide (EO) adducts thereof; diamines and ethylene oxide (EO) adducts thereof; polyamines and ethylene oxide (EO) adducts thereof; imidazolines; alkylglycines; benzalkonium chlorides; and the like.

The nonionic surfactant can be exemplified by ethylene oxide-adducted alkyl phenols, ethylene oxide-adducted higher alcohols, sucrose fatty acid esters, polyoxyethylene-adducted polyoxypropylene glycols, alkanolamine-fatty acid condensates, alkyl alkanolamides, polyoxyethylene alkyl ethers, polyoxyethylene polyoxypropylene alkyl ethers, polyoxyethylene polyoxypropylene glycols, alkyl glyceryl ethers, polyoxyethylene glycol fatty acid esters, polyoxyethylene hydrogenated castor oils, glycerin fatty acid esters, propylene glycol fatty acid esters, sorbitan fatty acid esters, polyoxyethylene sorbitan fatty acid esters, polyoxyethylene sorbitan tetraoleate, nonionic surfactants made by including a fluorinated hydrocarbon group in the above-mentioned nonionic surfactants, and the like.

The amphoteric surfactant can be exemplified by N-lauroylaminopropyl-N,N-dimethyl glycine, N-cocoylaminopropyl-N,N-dimethyl glycine, N-lauroylaminopropyl-N-carboxymethyl-N-hydroxylethyl glycine, N-oleylaminopropyl-N-carboxymethyl-N-hydroxylethyl glycine, N-3-dodecyloxy-2-hydroxypropyl-N,N-dimethyl glycine, N-cocoilaminopropyl-N-hydroxyethyl-3-amino-proprionic acid, tri-[3-(N-cocoilaminoethyl-N-hydroxyethyl-N-carboxymethyl)amino-2-hydroxypropanol]phosphate, and the like.

The oil agent can be exemplified by straight chain hydrocarbon oil agents, branched chain hydrocarbon oil agents, cyclic hydrocarbon oil agents, aromatic hydrocarbon oil agents, straight chain silicone oil agents, branched chain silicone oil agents, cyclic silicone oil agents, ester oil agents, glycol-based oil agents, carbitol-based oil agents, fluorinated hydrocarbon oil agents, and the like. In addition, these agents may or may not be volatile, and furthermore, they may or may not be soluble with water.

The alkali agent can be exemplified by ammonia, monoethanolamine (MEA), diethanolamine (DEA), triethanolamine (TEA), sodium hydroxide, potassium hydroxide, sodium carbonate, potassium carbonate, and the like.

The powder component can be exemplified by alumina, silica, kaoline, aluminum silicate, zeolite, bentonite, talc, montmorillonite, diatomaceous earth, cerium oxide, zirconium oxide, zirconium silicate, silicon carbide, titanium oxide, aluminum tripolyphosphate, aluminum hydroxide, barium sulfate, calcium silicate, sintered products thereof, and the like.

The viscosity modifier can be exemplified by polyvinyl alcohol (PVA), methylcellulose (MC), ethylcellulose, hydroxypropyl methylcellulose, hydroxypropyl ethylcellulose, and other cellulose derivatives, polyvinyl pyrrolidone (PVP), carboxymethyl cellulose, xanthan gum, alginic acid or salts thereof, carageenan, quince seed powder, *Alcaligenes*-produced polysaccharides, carboxyvinyl polymers, acrylic acid salts, acrylic acid polymers (chain type and crosslinked type), acrylic acid/methacrylic acid alkyl copolymers, and the like.
The gelling agent can be exemplified by glyceryl behenate/eicosadioate, polyglyceryl-10 behenate/eicosadioate, metal salts of fatty acids, hydroxystearic acid, dextrin fatty acid esters, inulin fatty acid esters, sucrose fatty acid esters, acylated cellobiose, dibenzylidene monosorbitol, amino acid-based gelling agents, silicic anhydride, organomodified clay mineral, fumed silica, alumina, crosslinked organopolysiloxane, hydrocarbon waxes such as a polyethylene wax and a paraffin wax, plant-based waxes such as a carnauba wax and a candelilla wax, agar, gelatin, and the like.

The sequestering agent can be exemplified by EDTA, NTA, DTPA, GLDA, HEDTA, GEDTA, TTHA, HIDA, DHEG, and the like.

The antiseptic agent can be exemplified by methyl parahydroxybenzoate, ethyl parahydroxybenzoate, butyl parahydroxybenzoate, propyl parahydroxybenzoate, phenoxyethanol, ethanol, and the like.

The perfume can be exemplified by camphor oil, orange oil, peppermint oil, jasmine absolute, pine oil, lime oil, lavender oil, rose oil, musk tincture, and the like.
The pH regulator can be exemplified by edetic acid, disodium edetate, sodium chloride, citric acid, sodium citrate, sodium hydroxide, potassium hydroxide, triethanolamine, and the like.
The pigment can be exemplified by Blue No. 1, Blue No. 204, Red No. 3, Yellow No. 201, and the like.

The application of the industrial use cleanser is not specifically limited, and a preferred example thereof is the application for washing and removing an oil soluble colorant. More specifically speaking, the industrial use cleanser can be preferably used for washing hands and fingers, washing coated wall surfaces, washing furniture, washing hard floor surfaces, washing glass, washing iron steel panels, washing coated surfaces of vehicles, and such activities.

The oil soluble colorant can be exemplified by cosmetics such as eye liner, mascara, eye shadow, eyebrow pencil, lipstick, lip gloss, lip balm, foundation cream, cheek color, manicure, and nail enamel; writing utensils such as oil marker pens, oil ballpoint pens, oil fountain pens, oil colors, oil paints, oil crayons; and the like.

By washing a surface to which the oil soluble colorant has been applied, with the industrial use cleanser serving as the sixth aspect of the present invention, it is possible to efficiently wash and remove the applied oil soluble colorant without eroding the surface.
In other words, if the industrial use cleanser of the sixth aspect of the present invention is used as an agent to remove an oil soluble colorant, the resulting product surpasses conventional types of industrial use cleansers in the cleansing properties (property to remove the oil soluble colorant) and the low corrosion properties (property of not damaging the surface applied with the oil soluble colorant).

If the industrial use cleanser including the 1,3-propanediol difatty acid ester is used as such an agent to remove an oil soluble colorant, the proportion of the content of the PDO-C8/C10 diester in the industrial use cleanser is preferably from 10 to 100% by mass, more preferably from 20 to 100% by mass, yet more preferably from 50 to 100% by mass, even yet more preferably from 80 to 100% by mass, and may also be 100% by mass.
By having the proportion of the content within the above-mentioned range, the cleansing properties and the low corrosion properties of the industrial use cleanser as an agent to remove an oil soluble colorant, can be more improved.

Moreover, if the industrial use cleanser including the 1,3-propanediol difatty acid ester is an industrial use cleanser that has been emulsified by including a surfactant (an emulsifying agent), the proportion of the content of the PDO-C8/C10 diester in the industrial use cleanser, when used as such an agent to remove an oil soluble colorant, is preferably from 10 to 100% by mass, more preferably from 20 to 90% by mass, and yet more preferably from 30 to 80% by mass.
By having the proportion of the content within the above-mentioned range, the cleansing properties and the low corrosion properties of the above-mentioned emulsified industrial use cleanser as an agent to remove an oil soluble colorant, can be more improved.

For example, it is difficult for a conventional type of industrial use cleanser, oil agent, or the like, to erase letters, paintings, or marks, painted on the coated surface of a car body, or the like, with an ink of a usual oil paint pen. This is because, if a conventional type of industrial use cleanser, oil agent, or the like, having a high property to remove such an oil soluble colorant, is used, the ink could be removed whereas the surface painted with the oil paint pen (for example, the coated surface of the car body) might be eroded.

However, if the industrial use cleanser of the sixth aspect of the present invention is used as such an agent to remove an ink of an oil paint pen, it is possible to efficiently remove the ink of the oil paint pen without eroding the surface painted with the oil paint pen.
In other words, if the industrial use cleanser including the 1,3-propanediol difatty acid ester of the sixth aspect of the present invention is used as an agent to remove an ink of an oil paint pen, the resulting product surpasses conventional types of industrial use cleansers and the like, in the cleansing properties (property to remove the ink) and the low corrosion properties (property of not damaging the surface painted with the oil paint pen).

If the industrial use cleanser including the PDO-C8/C10 diester is used as an agent to remove an ink of an oil paint pen, the proportion of the content of the PDO-C8/C10 diester in the industrial use cleanser is preferably from 10 to 100% by mass, more preferably from 20 to 100% by mass, yet more preferably from 50 to 100% by mass, even yet more preferably from 80 to 100% by mass, and may also be 100% by mass.
By having the proportion of the content within the above-mentioned range, the cleansing properties and the low corrosion properties of the industrial use cleanser, as an agent to remove an ink of an oil paint pen can be more improved.

Moreover, if the industrial use cleanser including the 1,3-propanediol difatty acid ester is an industrial use cleanser that has been emulsified by including a surfactant (an emulsifying agent), the proportion of the content of the PDO-C8/C10 diester in the industrial use cleanser, when used as such an agent to remove an ink of an oil paint pen, is preferably from 10 to 100% by mass, more preferably from 20 to 90% by mass, and yet more preferably from 30 to 80% by mass.
By having the proportion of the content within the above-mentioned range, the cleansing properties and the low corrosion properties of the above-mentioned emulsified industrial use cleanser, as an agent to remove an oil soluble colorant can be more improved.

The raw materials of the industrial use cleanser serving as the sixth aspect of the present invention are preferably derived from plant or microbial fermentation, and more preferably from plant. By not using a fossil fuel-derived material as a raw material of the industrial use cleanser, the load on the environment can be reduced. In addition, by using plant-derived materials as raw materials of the industrial use cleanser, the load on the environment can be much reduced.

The proportion of the plant-derived or microbial fermentation-derived raw materials in the entire raw materials of the industrial use cleanser serving as the sixth aspect of the present invention is preferably from 5 to 100% by mass, more preferably from 25 to 100% by mass, yet more preferably from 50 to 100% by mass, particularly preferably from 75 to 100% by mass, and most preferably 100% by mass (meaning that all the raw materials are substantially derived from plant or microbial fermentation).

In the method for producing the cosmetic preparation of the first aspect of the present invention serving as the seventh aspect of the present invention, a water-in-oil (W/O) emulsified cosmetic preparation is made by adding the 1,3-propanediol to a lipophilic solution in which the emulsifying agent, the 1,3-propanediol difatty acid ester, and, if required, an auxiliary ingredient, have been previously dissolved or dispersed.

In the lipophilic solution, if required, an auxiliary ingredient can be previously dissolved or dispersed. In this case, it is preferable to make the water-in-oil (W/O) emulsified cosmetic preparation by adding the 1,3-propanediol to a lipophilic solution in which the emulsifying agent, the 1,3-propanediol difatty acid ester, and the auxiliary ingredient have been previously dissolved or dispersed, so as to produce the cosmetic preparation serving as the first aspect of the present invention.

The method to prepare the lipophilic solution in which the emulsifying agent and the 1,3-propanediol difatty acid ester have been previously dissolved or dispersed is not specifically limited as long as these respective ingredients are not damaged by the method. For example, it is possible to prepare the lipophilic solution by mixing with these respective ingredients, and then subjecting the yielded solution or dispersion to a heat treatment, an ultrasonic treatment, a stirring treatment using a disperser, and/or the like. Of these, a method using heat and stirring treatments is preferred since the respective ingredients can be efficiently dissolved or dispersed. The auxiliary ingredient can be added to the lipophilic solution as required.

The auxiliary ingredient is not specifically limited as long as the effect of the present invention is not impaired. Preferred examples thereof can include the auxiliary ingredients which have been exemplified in the cosmetic preparation of the first aspect of the present invention, and the like.

The method to make such a water-in-oil (W/O) emulsified cosmetic preparation by adding the 1,3-propanediol to the lipophilic solution is not specifically limited, as long as the effect of the present invention is not impaired, and as long as the ingredients in the lipophilic solution and the 1,3-propanediol are not damaged. For example, the method is preferably such that the 1,3-propanediol that has been heated to 60 to 80°C is gradually added to the lipophilic solution that has been heated to 60 to 80°C under stirring, thereby making a mixed solution, and the mixed solution is cooled down to room temperature (from 20 to 25°C) under stirring so as to make the water-in-oil (W/O) emulsified cosmetic preparation.

It is also possible that the 1,3-propanediol is previously added to purified water, and then the yielded solution is subjected to a heat treatment, an ultrasonic treatment, a stirring treatment using a disperser, and/or the like, thereby preparing a hydrophilic solution, and the hydrophilic solution is added to the lipophilic solution.
The proportion of the content of purified water in the hydrophilic solution is not specifically limited as long as the effect of the present invention is not impaired. Although the proportion may be 100% by mass, the proportion is preferably from 1 to 99.5% by mass, more preferably from 1 to 95% by mass, and yet more preferably from 1 to 90% by mass. If purified water is contained within the above-mentioned range, the auxiliary ingredient can be added to the hydrophilic solution as required.

Regarding the method of adding one or some of these auxiliary ingredients that is(are) preferably kept unheated, to the cosmetic preparation, preferred is a method in which the auxiliary ingredient(s) is(are) not previously dissolved or dispersed in the lipophilic or hydrophilic solution, but the auxiliary ingredient(s) is(are) added thereto at the point of time when the mixed solution has been cooled down to an appropriate temperature during the process of lowering the temperature of the mixed solution to room temperature.

In the method for producing a cosmetic preparation serving as the eighth aspect of the present invention, a water-in-oil (W/O) emulsified cosmetic preparation is made by adding a hydrophilic solution in which, if required, an auxiliary ingredient has been previously dissolved or dispersed, to a lipophilic solution in which the cosmetic preparation composition of the third aspect of the present invention has been previously dissolved or dispersed.

The lipophilic solution is not specifically limited as long as the effect of the present invention is not impaired.
Moreover, it is preferable to use the lipophilic solution in such a way that the water-in-oil (W/O) emulsified cosmetic preparation is made by adding the hydrophilic solution, in which, if required, the auxiliary ingredient has been previously dissolved or dispersed, to the lipophilic solution in which the cosmetic preparation composition of the third aspect of the present invention has been previously dissolved or dispersed, so as to produce the cosmetic preparation serving as the fifth aspect of the present invention. In this case, if required, it is also possible to previously dissolve and disperse the auxiliary ingredient in a lipophilic solution in which the cosmetic preparation composition of the third aspect of the present invention has been dissolved or dispersed.

The method to make such a water-in-oil (W/O) emulsified cosmetic preparation by adding the hydrophilic solution, in which, if required, the auxiliary ingredient has been previously dissolved or dispersed, to the lipophilic solution in which the cosmetic preparation composition of the third aspect of the present invention has been previously dissolved or dispersed, is not specifically limited, as long as the effect of the present invention is not impaired, and as long as the cosmetic preparation composition of the third aspect of the present invention and the ingredients in the hydrophilic solution are not damaged. For example, the method is preferably such that the hydrophilic solution that has been heated to 60 to 80°C is gradually added to the cosmetic preparation composition of the third aspect of the present invention that has been heated to 60 to 80°C under stirring, thereby making a mixed solution, and the mixed solution is cooled down to room temperature (from 20 to 25°C) under stirring so as to make the water-in-oil (W/O) emulsified cosmetic preparation.

The method to prepare the lipophilic solution in which the cosmetic preparation composition of the third aspect of the present invention, and, if required, the auxiliary ingredient, have been previously dissolved or dispersed, is not specifically limited as long as the cosmetic preparation composition of the third aspect of the present invention and the auxiliary ingredient are not damaged by the method. For example, it is possible to prepare the lipophilic solution by mixing with the cosmetic preparation composition of the third aspect of the present invention and the auxiliary ingredient, and then subj ecting the mixture to a heat treatment, an ultrasonic treatment, a stirring treatment using a disperser, and/or the like. Of these, a method using a heat treatment is preferred since the cosmetic preparation composition of the third aspect of the present invention and the auxiliary ingredient can be efficiently dissolved or dispersed.

The method to prepare the hydrophilic solution in which, if required, the auxiliary ingredient has been previously dissolved or dispersed is not specifically limited as long as the auxiliary ingredient is not damaged. For example, it is possible to prepare the solution by adding the auxiliary ingredient to purified water, and then subjecting the yielded solution to a heat treatment, an ultrasonic treatment, a stirring treatment using a disperser, and/or the like. Of these, a method using a heat treatment is preferred since the auxiliary ingredient can be efficiently dissolved or dispersed.
The proportion of the content of purified water in the hydrophilic solution is not specifically limited as long as the effect of the present invention is not impaired, although the proportion is preferably from 1 to 99.5% by mass, more preferably from 1 to 95% by mass, and yet more preferably from 1 to 90% by mass. If purified water is contained within the above-mentioned range, the auxiliary ingredient can be added to the hydrophilic solution as required.

The auxiliary ingredient is not specifically limited as long as the effect of the present invention is not impaired. Preferred examples thereof can include the auxiliary ingredients, the emulsifying agents, and the 1,3-propanediol, which have been exemplified in the cosmetic preparation of the first aspect of the present invention, and the like.

Regarding the method of adding one or some of these auxiliary ingredients that is(are) preferably kept unheated, to the cosmetic preparation, preferred is a method in which the auxiliary ingredient(s) is(are) not previously dissolved or dispersed in the lipophilic solution or the hydrophilic solution, but the auxiliary ingredient(s) is(are) added thereto at the point of time when the mixed solution has been cooled down to an appropriate temperature during the process of lowering the temperature of the mixed solution to room temperature.

### EXAMPLES

Next is a more detailed description of the present invention with reference to Examples. However, the present invention is not to be limited to the following Examples.
The viscosity of the isostearic acid was measured by a stress-controlled rheometer (a product of Haake Company, RheoStress RS 1) using a rotor C60/2 Ti at 10°C.

[Synthesis Example 1: Synthesis of PDO diisostearate] Into a 1L four-neck flask equipped with a stirrer, a thermometer, a nitrogen gas inlet tube, and a moisture separator, were charged 79 g (1.04 mol) of microbial fermentation-derived 1,3-propanediol (Zemea Propanediol, a product of DuPont Tate & Lyle BioProducts), and 621 g (2.13 mol) of rape seed- or such plant-derived isostearic acid (Product Name: Emersol 875, a product of Cognis GmbH. having a viscosity of 103 mPa·s at 10°C with 10 Pa). The mixture was reacted under a nitrogen gas stream, while removing generated water, at 220°C for 9 hours. The excessive fatty acids were removed by reducing the pressure. Decolorization and deodorization were carried out. By so doing, 490 g (yield 70%) of PDO diisostearate was obtained.
The purity of the obtained PDO diisostearate was checked by gas chromatography (with a GC-2010 chromatography unit having a DB-5HT column and FID detector, a product of Shimadzu Corporation).
The thus obtained PDO diisostearate was used for the present invention.

[Synthesis Example 2: Synthesis of PDO-C8/C10 diester] Into a 1L four-neck flask equipped with a stirrer, a thermometer, a nitrogen gas inlet tube, and a moisture separator, were charged 130 g (1.71 mol) of microbial fermentation-derived 1,3-propanediol (Zemea Propanediol, a product of DuPont Tate & Lyle BioProducts), 407 g (2.83 mol) of palm kernel-derived n-octanoic acid (caprylic acid) (a product of Wako Pure Chemical Industries, Ltd.), and 163 g (0.92 mol) of palm kernel-derived n-decanoic acid (capric acid) (a product of Wako Pure Chemical Industries, Ltd.). The mixture was reacted under a nitrogen gas stream, while removing generated water, at 220°C for 7 hours. The excessive fatty acids were removed by reducing the pressure. Decolorization and deodorization were carried out. By so doing, 420 g (yield 60%) of PDO-C8/C10 diester was obtained.
The purity of the obtained PDO-C8/C10 diester was checked by gas chromatography (with a GC-2010 chromatography unit having a DB-5HT column and FID detector, a product of Shimadzu Corporation).
As a result, the mass ratio of the n-octanoic acid and the n-decanoic acid in terms of the fatty acids which constitute the PDO-C8/C10 diester was 3:1.

### [Synthesis Example 3: Synthesis of PDO-di(2-(4,4-dimethylpentan-2-yl)-5,7,7-trimethyloctanoate]

Into a 1L four-neck flask equipped with a stirrer, a thermometer, a nitrogen gas inlet tube, and a moisture separator, were charged 79 g (1.04 mol) of microbial fermentation-derived 1,3-propanediol (Zemea Propanediol, a product of DuPont Tate & Lyle BioProducts), and 621 g (2.13 mol) of isostearic acid comprising 2-(4,4-dimethylpentan-2-yl)-5,7,7-trimethyloctanoic acid as a main ingredient (hereunder, may be referred to as multibranched isostearic acid) (Product Name: Isostearic Acid, (a product of Nissan Chemical Industries, Ltd. having a viscosity of 8100 mPa·s at 10°C with 10 Pa)). The mixture was reacted under a nitrogen gas stream, while removing generated water, at 220°C for 9 hours. The excessive fatty acids were removed by reducing the pressure. Decolorization and deodorization were carried out. By so doing, 434 g (yield 62%) of PDO multibranched diisostearate was obtained.

### [Synthesis Example 4: Synthesis of PDO-Garbett isostearic acid diester]

Into a 1L four-neck flask equipped with a stirrer, a thermometer, a nitrogen gas inlet tube, and a moisture separator, were charged 79 g (1.04 mol) of microbial fermentation-derived 1,3-propanediol (Zemea Propanediol, a product of DuPont Tate & Lyle BioProducts), and 621 g (2.13 mol) of isostearic acid comprising 2-hexyl-dodecanoic acid and 2-heptyl-decanoic acid as main ingredients (hereunder, may be referred to as Garbett isostearic acid) (Product Name: Isostearic Acid T (a product ofNissan Chemical Industries, Ltd. having a viscosity of 98 mPa·s at 10°C with 10 Pa)). The mixture was reacted under a nitrogen gas stream, while removing generated water, at 220°C for 9 hours. The excessive fatty acids were removed by reducing the pressure. Decolorization and deodorization were carried out. By so doing, 455 g (yield 65%) of PDO diisostearate (PDO-Garbett isostearic acid diester) was obtained.

[Synthesis Example 5: Synthesis of PDO distearate] Into a 1L four-neck flask equipped with a stirrer, a thermometer, a nitrogen gas inlet tube, and a moisture separator, were charged 79 g (1.04 mol) of microbial fermentation-derived 1,3-propanediol (Zemea Propanediol, a product of DuPont Tate & Lyle BioProducts), and 621 g (2.13 mol) of stearic acid (a product of Wako Pure Chemical Industries, Ltd.). The mixture was reacted under a nitrogen gas stream, while removing generated water, at 220°C for 9 hours. The excessive fatty acids were removed by reducing the pressure. Decolorization and deodorization were carried out. By so doing, 455 g (yield 65%) of PDO distearate was obtained.

[Synthesis Example 6: Synthesis of PDO-C8 diester (PDO-di-n-octanoic acid ester)] Into a 1L four-neck flask equipped with a stirrer, a thermometer, a nitrogen gas inlet tube, and a moisture separator, were charged 76 g (1.00 mol) of microbial fermentation-derived 1,3-propanediol (Zemea Propanediol, a product of DuPont Tate & Lyle BioProducts), and 302.4 g (2.10 mol) of palm kernel-derived n-octanoic acid (caprylic acid) (a product of Wako Pure Chemical Industries, Ltd.). The mixture was reacted under a nitrogen gas stream, while removing generated water, at 200°C for 5 hours. The excessive fatty acids were removed by reducing the pressure. Decolorization and deodorization were carried out. By so doing, 275 g (yield 82.8%) of PDO-C8 diester was obtained.
The purity of the obtained PDO-C8 diester was checked by gas chromatography (with a GC-2010 chromatography unit having a DB-5HT column and FID detector, a product of Shimadzu Corporation).

[Synthesis Example 7: Synthesis of PDO-C8/C10 diester] Into a 1L four-neck flask equipped with a stirrer, a thermometer, a nitrogen gas inlet tube, and a moisture separator, were charged 76 g (1.00 mol) of microbial fermentation-derived 1,3-propanediol (Zemea Propanediol, a product of DuPont Tate & Lyle BioProducts), 172.8 g (1.2 mol) of palm kernel-derived n-octanoic acid (caprylic acid) (a product of Wako Pure Chemical Industries, Ltd.), and 206.4 g (1.2 mol) of palm kernel-derived n-decanoic acid (capric acid) (a product of Wako Pure Chemical Industries, Ltd.). The mixture was reacted under a nitrogen gas stream, while removing generated water, at 220°C for 7 hours. The excessive fatty acids were removed by reducing the pressure. Decolorization and deodorization were carried out. By so doing, 290 g (yield 81.5%) of PDO-C8/C10 diester was obtained.
The purity of the obtained PDO-C8/C10 diester was checked by gas chromatography (with a GC-2010 chromatography unit having a DB-5HT column and FID detector, a product of Shimadzu Corporation). As a result, the mass ratio of the n-octanoic acid and the n-decanoic acid in terms of the fatty acids which constitute the PDO-C8/C10 diester was 1:1.

[Synthesis Example 8: Synthesis of PDO-C8/C10 diester] Into a 1L four-neck flask equipped with a stirrer, a thermometer, a nitrogen gas inlet tube, and a moisture separator, were charged 76 g (1.00 mol) of microbial fermentation-derived 1,3-propanediol (Zemea Propanediol, a product of DuPont Tate & Lyle BioProducts), 79.2 g (0.55 mol) of palm kernel-derived n-octanoic acid (caprylic acid) (a product of Wako Pure Chemical Industries, Ltd.), and 283.8 g (1.65 mol) of palm kernel-derived n-decanoic acid (capric acid) (a product of Wako Pure Chemical Industries, Ltd.). The mixture was reacted under a nitrogen gas stream, while removing generated water, at 220°C for 7 hours. The excessive fatty acids were removed by reducing the pressure. Decolorization and deodorization were carried out. By so doing, 295 g (yield 82.9%) of PDO-C8/C10 diester was obtained.
The purity of the obtained PDO-C8/C10 diester was checked by gas chromatography (with a GC-2010 chromatography unit having a DB-5HT column and FID detector, a product of Shimadzu Corporation). As a result, the mass ratio of the n-octanoic acid and the n-decanoic acid in terms of the fatty acids which constitute the PDO-C8/C10 diester was 1:3.

[Synthesis Example 9: Synthesis of PDO-C10 diester (PDO-di-n-decanoic acid ester)] Into a 1L four-neck flask equipped with a stirrer, a thermometer, a nitrogen gas inlet tube, and a moisture separator, were charged 76 g (1.00 mol) of microbial fermentation-derived 1,3-propanediol (Zemea Propanediol, a product of DuPont Tate & Lyle BioProducts), and 361.2 g (2.10 mol) of palm kernel-derived palm kernel-derived n-decanoic acid (capric acid) (a product of Wako Pure Chemical Industries, Ltd.). The mixture was reacted under a nitrogen gas stream, while removing generated water, at 220°C for 7 hours. The excessive fatty acids were removed by reducing the pressure. Decolorization and deodorization were carried out. By so doing, 295 g (yield 76.0%) of PDO-C10 diester was obtained.
The purity of the obtained PDO-C10 diester was checked by gas chromatography (with a GC-2010 chromatography unit having a DB-5HT column and FID detector, a product of Shimadzu Corporation).

[Examples 1 to 5 and 1A to 1K, and Comparative Examples 1 and 1A to 1C] In Examples 1 to 5 and 1A to 1C, the PDO-C8/C10 diester obtained from Synthesis Example 2, hydrogenated soybean-derived lecithin (Product Name: Basis LS-60HR, a product of the Nisshin OilliO Group, Ltd.), palm-derived glycerin (a product of Sakamoto Yakuhin Kogyo Co., Ltd.), microbial fermentation-derived 1,3-propanediol (Product Name: Zemea Propanediol, a product of DuPont Tate & Lyle BioProducts), purified water, and palm-derived or palm kernel-derived polyglyceryl laurate (the average degree of polymerization of polyglycerin was 10, and the saponification value was from 35 to 75) (Product Name: Sun Soft Q-12S, a product of Taiyo Kagaku Co., Ltd.) were respectively mixed in accordance with the parts by mass as shown in Table 1 to Table 4, thereby preparing an ingredient A and an ingredient B.
In Examples 1D to 1K, the PDO-C8/C10 diester obtained from Synthesis Example 2, 7, or 8, the PDO-C8 diester obtained from Synthesis Example 6, or the PDO-C10 diester obtained from Synthesis Example 9, was used.
Moreover, in Comparative Examples 1 and 1A to 1C, fossil fuel-derived isononyl isononanoate (Product Name: Salacos 99, a product of the Nisshin OilliO Group, Ltd.) was used instead of the above-mentioned PDO-C8/C10 diester.

### <Amount of CO₂ (kg) emitted to the environment upon burning of 1t of cosmetic preparation>

The amount of CO₂ emitted to the environment upon burning of 1t of the cosmetic preparation was calculated based on the fossil fuel-derived raw material in accordance with the following equation (in view of the carbon neutral concept, the amount of CO₂ emitted from plant-derived and microorganism-derived raw materials was deemed to be ±0).
Amount of CO₂ (kg) emitted to the environment upon burning of 1t of cosmetic preparation = (Quantity of mixed fossil fuel-derived raw material/Molecular weight of the raw material) x Carbon number of the raw material x 44 (Molecular weight of carbon dioxide)

Next, the ingredient B that had been heated and melted at 80°C was charged into a homodisper (Product Name: T. K. Agihomomixer 2M-05 type, a product of Primix Corporation), to which the ingredient A that had been heated at 80°C was gradually added under stirring by means of the rotation of a disper blade at 5000 rpm. On completion of the addition, the rotation speed of the disper blade of the stirrer was slowed down to 1500 rpm. The mixture was cooled down to room temperature. By so doing, cleansing gels were obtained.

The cleansing properties and the low irritation properties of the obtained cleansing gels were evaluated in the following manners.
The insides of the arms of seven panelists were washed with soap and water, and in addition, the washed surfaces were wiped with ethanol and naturally dried. Thereafter, using a cosmetic preparation including silicones (lipstick) (Product Name: Hydrabase No. 23, a product of Chanel), a line was painted respectively on the washed surfaces of the insides of their arms. Next, the painted line was evenly divided into twenty equal areas. Each area was rubbed with an absorbent cotton that had been impregnated with, but to a degree which would not allow a leakage of, the cleansing gel of Examples 1 to 5 and 1A to 1C, or Comparative Examples 1 and 1A to 1C, while loading a pressure of about 200 g in a reciprocating manner thirty times. From the proportion of the line of the above-mentioned silicone-including cosmetic preparation that was able to be washed off by this cleansing operation, the cleansing properties of the respective cleansing gels were evaluated in accordance with the following criteria. The results of their averages are shown in Table 1 to Table 4.
Moreover, during the cleansing process mentioned above, the low irritation properties of the respective cleansing gels were evaluated in accordance with the following criteria. The results of their averages are shown in Table 1 to Table 4.

### <Evaluation criteria of cleansing properties>

Excellent: High cleansing properties, meaning that a proportion of 75 to 100% in the line painted with the silicone-including cosmetic preparation on the skin was able to be washed off.
Good: Relatively high cleansing properties, meaning that a proportion of 50 to 75% in the line painted with the silicone-including cosmetic preparation on the skin was able to be washed off.
Moderate: Some cleansing properties, meaning that only a proportion of 25 to 50% in the line painted with the silicone-including cosmetic preparation on the skin was able to be washed off.
Poor: Weak cleansing properties, meaning that only a proportion of 0 to 25% in the line painted with the silicone-including cosmetic preparation on the skin was able to be washed off.

### <Evaluation criteria of low irritation properties>

A: Almost no tingling irritation was felt.
B: Tingling irritation was felt.

**[Table 1]**

| Cleansing gel | | | | | | | |
|---|---|---|---|---|---|---|---|
| Ingredients | | Comparative Example 1 | Example 1 | Example 2 | Example 3 | Example 4 | Example 5 |
| A | PDO-C8/C10 diester | 0 | 56.0 | 55.9 | 55.5 | 55.0 | 53.0 |
| | Isononyl isononanoate | 56.0 | 0 | 0 | 0 | 0 | 0 |
| B | Hydrogenated lecithin | 1.0 | 1.0 | 1.0 | 1.0 | 1.0 | 1.0 |
| | Glycerin | 30.0 | 30.0 | 30.0 | 30.0 | 30.0 | 30.0 |
| | 1,3-propanediol | 10.0 | 10.0 | 10.0 | 10.0 | 10.0 | 10.0 |
| | Purified water | 3.0 | 3.0 | 3.0 | 3.0 | 3.0 | 3.0 |
| | Polyglyceryl-10 laurate | 0 | 0 | 0.1 | 0.5 | 1.0 | 3.0 |
| Amount of CO₂ (kg) emitted to the environment upon burning of 1t | | 156.2 | ±0 | ±0 | ±0 | ±0 | ±0 |
| Cleansing properties | | Good | Good | Good | Excellent | Excellent | Excellent |
| Low irritation properties | | B | A | A | A | A | A |

**[Table 2]**

| Cleansing gel | | | | | | | |
|---|---|---|---|---|---|---|---|
| Ingredients | | Example 1A | Example 1B | Example 1C | Comparative Example 1A | Comparative Example 1B | Comparative Example 1C |
| A | PDO-C8/C10 diester | 5.0 | 10.0 | 25.0 | 0 | 0 | 0 |
| | Isononyl isononanoate | 0 | 0 | 0 | 5.0 | 10.0 | 25.0 |
| B | Hydrogenated lecithin | 1.0 | 1.0 | 1.0 | 1.0 | 1.0 | 1.0 |
| | Glycerin | 45.5 | 43.0 | 35.5 | 45.5 | 43.0 | 35.5 |
| | 1,3-propanediol | 45.5 | 43.0 | 35.5 | 45.5 | 43.0 | 35.5 |
| | Purified water | 3.0 | 3.0 | 3.0 | 3.0 | 3.0 | 3.0 |
| | Polyglycelyl-10 laurate | 0 | 0 | 0 | 0 | 0 | 0 |
| Amount of CO₂ (kg) emitted to the environment upon burning of 1t | | ±0 | ±0 | ±0 | 13.9 | 27.9 | 69.7 |
| Cleansing properties | | Excellent | Excellent | Excellent | Moderate | Good | Good |
| Low irritation properties | | A | A | A | A | B | B |

**[Table 3]**

| Cleansing gel | | | | | |
|---|---|---|---|---|---|
| Ingredients | | Example 1D | Example 1E | Example 1F | Example 1G |
| A | PDO-C8/C10 = 75:25 diester | 0 | 0 | 0 | 0 |
| | PDO-C8 diester | 5.0 | 56.0 | 0 | 0 |
| | PDO-C8/C10 = 50:50 diester | 0 | 0 | 5.0 | 56.0 |
| | PDO-C8/C10 = 25:75 diester | 0 | 0 | 0 | 0 |
| | PDO-C10 diester | 0 | 0 | 0 | 0 |
| | Isononyl isononanoate | 0 | 0 | 0 | 0 |
| B | Hydrogenated lecithin | 1.0 | 1.0 | 1.0 | 1.0 |
| | Glycerin | 45.5 | 30.0 | 45.5 | 30.0 |
| | 1,3-propanediol | 45.5 | 10.0 | 45.5 | 10.0 |
| | Purified water | 3.0 | 3.0 | 3.0 | 3.0 |
| | Polyglyceryl-10 laurate | 0 | 0 | 0 | 0 |
| Amount of CO₂ (kg) emitted to the environment upon burning of 1t | | ±0 | ±0 | ±0 | ±0 |
| Cleansing properties | | Excellent | Excellent | Good | Excellent |
| Low irritation properties | | B | B | A | A |

**[Table 4]**

| Cleansing gel | | | | | |
|---|---|---|---|---|---|
| Ingredients | | Example 1H | Example 1I | Example 1J | Example 1K |
| A | PDO-C8/C10 diester | 0 | 0 | 0 | 0 |
| | PDO-C8 diester | 0 | 0 | 0 | 0 |
| | PDO-C8/C10 = 50:50 diester | 0 | 0 | 0 | 0 |
| | PDO-C8/C10 = 25:75 diester | 5.0 | 56.0 | 0 | 0 |
| | PDO-C10 diester | 0 | 0 | 5.0 | 56.0 |
| | Isononyl isononanoate | 0 | 0 | 0 | 0 |
| B | Hydrogenated lecithin | 1.0 | 1.0 | 1.0 | 1.0 |
| | Glycerin | 45.5 | 30.0 | 45.5 | 30.0 |
| | 1,3-propanediol | 45.5 | 10.0 | 45.5 | 10.0 |
| | Purified water | 3.0 | 3.0 | 3.0 | 3.0 |
| | Polyglyceryl-10 laurate | 0 | 0 | 0 | 0 |
| Amount of CO₂ (kg) emitted to the environment upon burning of 1t | | ±0 | ±0 | ±0 | ±0 |
| Cleansing properties | | Moderate | Good | Poor | Poor |
| Low irritation properties | | A | A | A | A |

From the above-mentioned results, it is apparent that the cleansing gels of Examples 1 to 5, 1A to 1C, 1F, 1G, and 1I according to the present invention have equivalent or better cleansing properties than those of the cleansing gels of Comparative Examples 1 and 1A to 1C, as well as having excellent low irritation properties. Moreover, since all the raw materials (except for purified water) of the cleansing gels of Examples 1 to 5 and 1A to 1C are derived from plant or microbial fermentation, the load on the environment is reduced as compared to the cases of the cleansing gels of Comparative Examples 1 and 1A to 1C, as apparent from the amount of CO₂ emitted to the environment (kg) upon burning of 1t.

[Examples 6 to 8 and 6A to 6D, and Comparative Examples 2 and 2A to 2C] In Examples 6 to 8 and 6A to 6D, the PDO diisostearate obtained from Synthesis Example 1, hydrogenated soybean-derived lecithin (Product Name: Basis LS-60HR, a product of the Nisshin OilliO Group, Ltd.), palm-derived glycerin (a product of Sakamoto Yakuhin Kogyo Co., Ltd.), microbial fermentation-derived 1,3-propanediol (Product Name: Zemea Propanediol, a product of DuPont Tate & Lyle BioProducts), and purified water were respectively mixed in accordance with the parts by mass as shown in Table 5 and Table 6, thereby preparing an ingredient A and an ingredient B.
Moreover, in Comparative Examples 2 and 2A to 2C, a fossil fuel-derived liquid paraffin (a product ofNacalai Tesque, Inc.), the PDO multibranched diisostearate obtained from Synthesis Example 3, the PDO-Garbett isostearic acid diester obtained from Synthesis Example 4, or a refined macadamia nut oil (a product of Yokozeki Oil & Fat Industries Co., Ltd.), were respectively used instead of the above-mentioned PDO diisostearate.

Next, the ingredient B that had been heated and melted at 80°C was charged into a homodisper (Product Name: T. K. Agihomomixer 2M-05 type, a product of Primix Corporation), to which the ingredient A that had been heated at 80°C was gradually added under stirring by means of the rotation of a disper blade at 5000 rpm. On completion of the addition, the rotation speed of the disper blade of the stirrer was slowed down to 1500 rpm. The mixture was cooled down to room temperature. By so doing, moisturizing gels were obtained.

The moisturizing properties and the feeling of use of the obtained moisturizing gels were evaluated in the following manners.
The insides of the arms of seven panelists were washed with soap and water, and in addition, the washed surfaces were wiped with ethanol and naturally dried. Thereafter, each of the thus washed surfaces was evenly divided into eleven equal areas, to which 0.05 g of the respective moisturizing gels of Examples 6 to 8 and 6A to 6D, and Comparative Examples 2 and 2A to 2C, was applied. The area was gently rubbed with a finger ten times, and then wiped with a dry cloth. On completion of this wiping, the moisturizing properties, the feeling of use, and the stability of the respective moisturizing gels were evaluated in accordance with the following criteria. The results of their averages are shown in Table 5 and Table 6.

### <Evaluation criteria of moisturizing properties>

Good: Very soft and moistened touch was felt.
Moderate: Soft and moistened touch was felt.
Poor: No moistened touch was felt.

### <Evaluation criteria of feeling of use>

Good: No sticky texture was felt.
Moderate: Slightly sticky texture was felt.
Poor: Sticky texture was felt.

### <Evaluation criteria of stability>

Good: No odor was noticed after a week storage at 50°C.
Poor: An odor was noticed after a week storage at 50°C.

**[Table 5]**

| Moisturizing gel | | | | | |
|---|---|---|---|---|---|
| Ingredients | | Comparative Example 2 | Example 6 | Example 7 | Example 8 |
| A | PDO diisostearate | 0 | 56.0 | 34.0 | 20.8 |
| | Liquid paraffin | 56.0 | 0 | 0 | 0 |
| B | Hydrogenated lecithin | 1.0 | 1.0 | 1.5 | 1.8 |
| | Glycerin | 30.0 | 30.0 | 45.0 | 54.0 |
| | 1,3-propanediol | 10.0 | 10.0 | 15.0 | 18.0 |
| | Purified water | 3.0 | 3.0 | 4.5 | 5.4 |
| Moisturizing properties | | Poor | Good | Good | Good |
| Feeling of use | | Good | Good | Good | Good |
| Stability | | Good | Good | Good | Good |

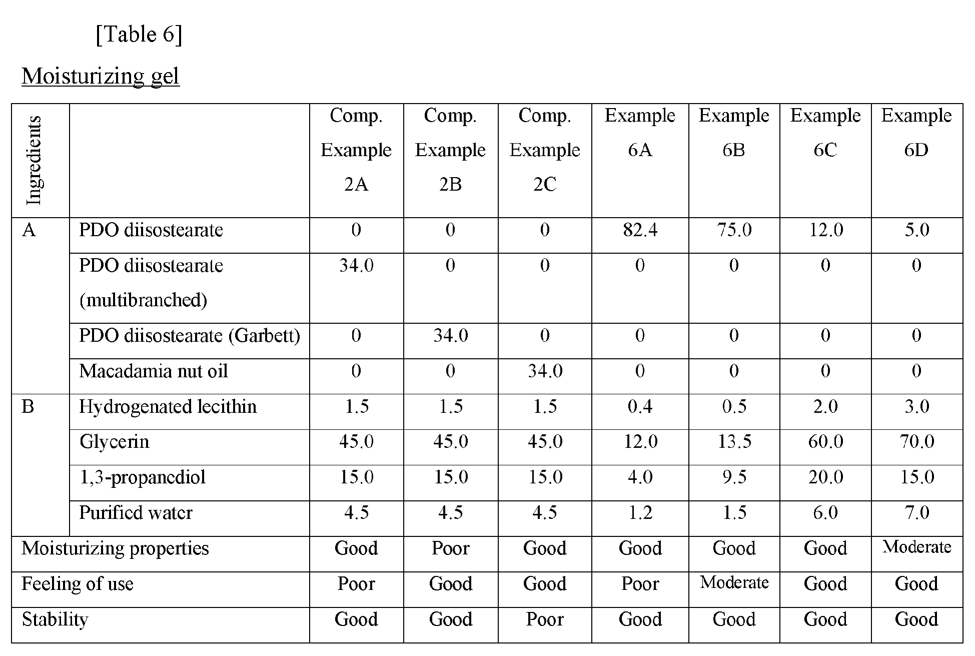

From the above-mentioned results, it is apparent that the moisturizing gels of Examples 6 to 8 and 6A to 6D according to the present invention have equivalent moisturizing properties to those of the moisturizing gel of Comparative Example 2, as well as having excellent feeling of use. Moreover, since all the raw materials (except for purified water) of the moisturizing gels of Examples 6 to 8 and 6A to 6D are derived from plant or microbial fermentation, the load on the environment is reduced as compared to the case of the moisturizing gel of Comparative Example 2.
Furthermore, it is apparent that the moisturizing gels of Examples 6 to 8 and 6A to 6D according to the present invention have equivalent moisturizing properties and/or feeling of use to those of the case where a macadamia nut oil is used, as well as having higher stability than this case.

[Examples 9 to 11 and 9Ato 9D, and Comparative Examples 3 and 3Ato 3C] In Examples 9 to 11 and 9A to 9D, the PDO diisostearate obtained from Synthesis Example 1, hydrogenated soybean-derived lecithin (Product Name: Basis LS-60HR, a product of the Nisshin OilliO Group, Ltd.), palm-derived glycerin (a product of Sakamoto Yakuhin Kogyo Co., Ltd.), microbial fermentation-derived 1,3-propanediol (Product Name: Zemea Propanediol, a product of DuPont Tate & Lyle BioProducts), purified water, and a quince seed powder (Product Name: Quince Seed Powder, a product of Taiyo Kagaku Co., Ltd.) were respectively mixed in accordance with the parts by mass as shown in Table 7 and Table 8, thereby preparing an ingredient A, an ingredient B, and an ingredient C.
Moreover, in Comparative Examples 3 and 3A to 3C, a fossil fuel-derived liquid paraffin (a product ofNacalai Tesque, Inc.), the PDO multibranched diisostearate obtained from Synthesis Example 3, the PDO-Garbett isostearic acid diester obtained from Synthesis Example 4, or a refined macadamia nut oil (a product of Yokozeki Oil & Fat Industries Co., Ltd.), were respectively used instead of the above-mentioned PDO diisostearate.

Next, the ingredient B that had been heated and melted at 80°C was charged into a homodisper (Product Name: T. K. Agihomomixer 2M-05 type, a product of Primix Corporation), to which the ingredient A that had been heated at 80°C was gradually added under stirring by means of the rotation of a disper blade at 5000 rpm. On completion of the addition, the rotation speed of the disper blade of the stirrer was slowed down to 1500 rpm. At a point of time when the mixture was cooled down to 40°C, the ingredient C was added. The mixture was cooled down to room temperature. By so doing, moisturizing emulsions in forms of milky white turbid liquid (moisturizing cosmetic preparations) were obtained.

The moisturizing properties, the feeling of use, and the stability of the obtained moisturizing emulsions were evaluated in accordance with the same criteria as those of the moisturizing gels of Examples 6 to 8 mentioned above. The results of their averages are shown in Table 7 and Table 8.

**[Table 7] Moisturizing emulsion**

| Ingredients | | Comparative Example 3 | Example 9 | Example 10 | Example 11 |
|---|---|---|---|---|---|
| A | PDO diisostearate | 0 | 5.6 | 11.2 | 16.8 |
| | Liquid paraffin | 16.8 | 0 | 0 | 0 |
| B | Hydrogenated lecithin | 0.3 | 0.1 | 0.2 | 0.3 |
| | Glycerin | 9.0 | 3.0 | 6.0 | 9.0 |
| | 1,3-propanediol | 3.0 | 1.0 | 2.0 | 3.0 |
| | Purified water | 0.9 | 0.3 | 0.6 | 0.9 |
| C | 1,3-propanediol | 6.0 | 7.7 | 6.9 | 6.0 |
| | Quince seed powder | 0.3 | 0.4 | 0.3 | 0.3 |
| | Purified water | 63.7 | 81.9 | 72.8 | 63.7 |
| Moisturizing properties | | Poor | Good | Good | Good |
| Feeling of use | | Good | Good | Good | Good |
| Stability | | Good | Good | Good | Good |

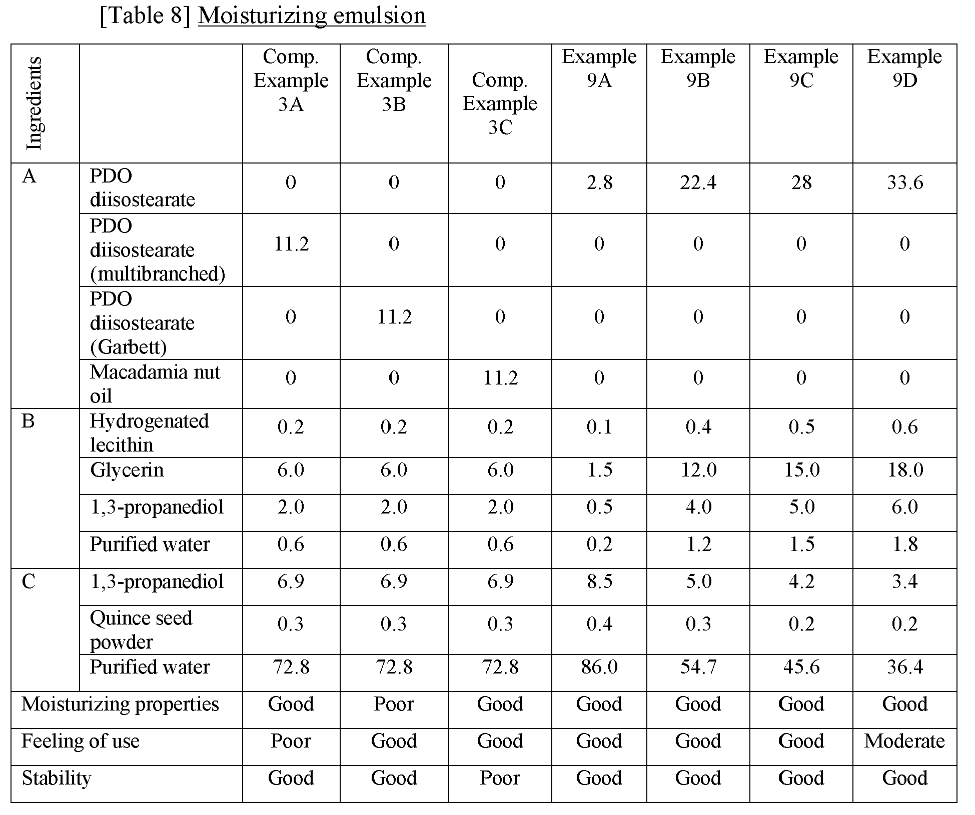

From the above-mentioned results, it is apparent that the moisturizing emulsions of Examples 9 to 11 and 9A to 9D according to the present invention have equivalent moisturizing properties to those of the moisturizing emulsion of Comparative Example 3, as well as having excellent feeling of use. Moreover, since all the raw materials (except for purified water) of the moisturizing emulsions of Examples 9 to 11 and 9A to 9D are derived from plant or microbial fermentation, the load on the environment is reduced as compared to the case of the moisturizing emulsion of Comparative Example 3.
Furthermore, it is apparent that the moisturizing emulsions of Examples 9 to 11 and 9A to 9D according to the present invention have equivalent moisturizing properties and/or feeling of use to those of the case where a macadamia nut oil is used, as well as having higher stability than this case.

[Example 12 and Comparative Examples 4 to 7] In Example 12, the PDO diisostearate obtained from Synthesis Example 1 was used as a cosmetic preparation composition (moisturizing ingredient) for a moisturizing cosmetic preparation (massage oil), and the moisturizing properties and the feeling of use thereof were evaluated.
Regarding Comparative Examples 4 to 7, a refined macadamia nut oil (a product of Yokozeki Oil & Fat Industries Co., Ltd.) was used in Comparative Example 4, squalane (a product of Wako Pure Chemical Industries, Ltd.) was used in Comparative Example 5, a meadowfoam oil (a product of Natural Plant Products LCC) was used in Comparative Example 6, and hydrogenated polydecene (a product of the Nisshin OilliO Group, Ltd.) was used in Comparative Example 7.
As additional Comparative Examples, the PDO distearate obtained from Synthesis Example 5 was used in Comparative Example 4A, the PDO multibranched diisostearate obtained from Synthesis Example 3 was used in Comparative Example 4B, and the PDO-Garbett isostearic acid diester obtained from Synthesis Example 4 was used in Comparative Example 4C.

The insides of the arms of seven panelists were washed with soap and water, and in addition, the washed surfaces were wiped with ethanol and naturally dried. Thereafter, each of the thus washed surfaces was evenly divided into eight equal areas, to which 0.05 g of the respective cosmetic preparation compositions of Example 12, and Comparative Examples 4 to 7 and 4A to 4C, was applied and massage was done by gently rubbing with a finger twenty times. Thereafter, the area was wiped with a dry cloth. On completion of this wiping, the moisturizing properties and the feeling of use of the respective cosmetic preparation compositions were evaluated in accordance with the same criteria as those of the moisturizing gels of Examples 6 to 8 mentioned above.
The results of their averages are shown in Table 9 and Table 10.

**[Table 9]**

| Cosmetic preparation composition for moisturizing cosmetic preparation | | | | | |
|---|---|---|---|---|---|
| | Comparative Example 4 | Comparative Example 5 | Comparative Example 6 | Comparative Example 7 | Example 12 |
| Moisturizing properties | Good | Good | Good | Good | Good |
| Feeling of use | Good | Good | Good | Good | Good |

**[Table 10]**

| Cosmetic preparation composition for moisturizing cosmetic preparation | | | |
|---|---|---|---|
| | Comparative Example 4A | Comparative Example 4B | Comparative Example 4C |
| Moisturizing properties | Moderate | Good | Poor |
| Feeling of use | Poor | Poor | Good |

From the above-mentioned results, it is apparent that the cosmetic preparation composition of Example 12 according to the present invention has equivalent or better moisturizing properties and feeling of use than those of the cosmetic preparation compositions of Comparative Examples 4 to 7 and 4A to 4C. In other words, it is apparent that the cosmetic preparation composition including the PDO diisostearate according to the present invention is suitable for the application as a substitute for a cosmetic preparation composition including a macadamia nut oil, for example, a moisturizing ingredient of a massage oil.

Next, the viscosity and the friction coefficient of the cosmetic preparation compositions of Example 12 and Comparative Examples 4 to 7 and 4A to 4C were measured.
The viscosity was measured by using a stress-controlled rheometer (Product No. RheoStress RS 1, with a C60/2 Ti sensor, a product of Haake Company) at 20°C.
The friction coefficient was measured by such a way that: 5 µL of each cosmetic preparation composition was applied to the surface of an artificial leather (Product Name: Supplale, a product of Idemitsu Technofine Co., Ltd.), and the applied surface was measured for the friction coefficient and the change in the value of the friction coefficient, by using a friction tester (Type: TL201Ts, a product of Trinity-Lab Co., Ltd.) with a pressure of 100 g. The results are shown in Table 11 and Table 12.

**[Table 11]**

| Cosmetic preparation composition for moisturizing cosmetic preparation | | | | | |
|---|---|---|---|---|---|
| | Comparative Example 4 | Comparative Example 5 | Comparative Example 6 | Comparative Example 7 | Example 12 |
| Viscosity (mPa·s) (20°C) | 82.0 | 36.0 | 105.4 | 98.0 | 80.0 |
| Friction coefficient | 0.138 | 0.155 | 0.134 | 0.130 | 0.140 |
| Changed value of friction coefficient | 0.0043 | 0.0062 | 0.0042 | 0.0050 | 0.0045 |

**[Table 12]**

| Cosmetic preparation composition for moisturizing cosmetic preparation | | | |
|---|---|---|---|
| | Comparative Example 4A | Comparative Example 4B | Comparative Example 4C |
| Viscosity (mPa·s)(20°C) | Not measurable | 1003 | 57.5 |
| Friction coefficient | - | 0.170 | 0.147 |
| Changed value of friction coefficient | - | 0.0080 | 0.0056 |

From the results of Table 11, it is apparent that the viscosity, the friction coefficient, and the changed value of the friction coefficient of the cosmetic preparation composition of Example 12 are quite similar to the viscosity, the friction coefficient, and the changed value of the friction coefficient of the cosmetic preparation composition of Comparative Example 4. In other words, the cosmetic preparation composition including the PDO diisostearate according to the present invention is quite similar to a cosmetic preparation composition including a macadamia nut oil, in these physical properties (the viscosity, the friction coefficient, and the change in the value of the friction coefficient). Accordingly, it is apparent that the cosmetic preparation composition including the PDO diisostearate according to the present invention can serve as a substitute for a cosmetic preparation composition including a macadamia nut oil, for the application as a skin-care cosmetic preparation such as a massage oil. In addition, because the PDO distearate of Comparative Example 4A was solid, the measurements of the viscosity and the friction coefficient were not feasible.

Furthermore, the oxidative stability was compared between the cosmetic preparation compositions of Example 12 and Comparative Example 4. Specifically speaking, these cosmetic preparation compositions were evaluated for the oxidative stability by a conductometric determination method (CDM method). The conductivity (µS/cm) measured by the CDM method shows higher value as the oxidization of the cosmetic preparation composition is more advanced. A greater increase in the conductivity per unit time means a lower oxidative stability of the cosmetic preparation composition. Conversely, a smaller increase in the conductivity per unit time means a higher oxidative stability of the cosmetic preparation composition. The conductivity was measured by using a rancimat instrument (Type: Rancimat 743, a product of Metrohm-Sibata Ltd.) in a condition where 3 g of the sample was examined under heating at 120°C with an air flow rate of 8 L/h. The results are shown in Table 13.

**[Table 13]**

| Cosmetic preparation composition for moisturizing cosmetic preparation | | | | |
|---|---|---|---|---|
| Elapsed time after initiation of measurement | After 5 hours | After 10 hours | After 15 hours | After 20 hours |
| Example 12 | 17 µS/cm | 45 µS/cm | 83 µS/cm | 127 µS/cm |
| Comparative Example 4 | 42 µS/cm | 161 µS/cm | 250 µS/cm | Over measurable limit |

From the results of Table 13, it is apparent that the cosmetic preparation composition of Example 12 has higher oxidative stability than that of the cosmetic preparation composition of Comparative Example 4. In other words, it is apparent that the cosmetic preparation composition including the PDO diisostearate according to the present invention has better oxidative stability than that of a cosmetic preparation composition including a macadamia nut oil.

### [Examples 13 and 13A to 13D, and Comparative Examples 8 and 9]

In Example 13, the cleansing properties and the low irritation properties of the PDO-C8/C10 diester obtained from Synthesis Example 2 as a cosmetic preparation composition for a cosmetic preparation to be used for the purpose of removing an ink of an oil paint pen (ink remover ingredient) from the skin, were evaluated.
In Examples 13A to 13D, the PDO-C8 diester obtained from Synthesis Example 6, the PDO-C8/C10 = 50:50 diester obtained from Synthesis Example 7, the PDO-C8/C10 = 25:75 diester obtained from Synthesis Example 8, or the PDO-C10 diester obtained from Synthesis Example 9, were respectively used instead of the PDO-C8/C10 diester obtained from Synthesis Example 2, and the cleansing properties and the low irritation properties thereof were evaluated upon removal of an ink of an oil paint pen from the skin, in the same manners as those of Example 13.
Regarding Comparative Examples 8 and 9, fossil fuel-derived isononyl isononanoate (a product of the Nisshin OilliO Group, Ltd.) was used in Comparative Example 8, and a liquid paraffin (a product of Nacalai Tesque, Inc.) was used in Comparative Example 9.

The insides of the arms of seven panelists were washed with soap and water, and in addition, the washed surfaces were wiped with ethanol and naturally dried. Thereafter, using a black oil paint pen (Product Name: Mckee Care, a product of Zebra Co., Ltd.), a line was painted respectively on the washed surfaces of the insides of their arms. Next, the painted line was evenly divided into seven equal areas. Each area was rubbed with an absorbent cotton that had been impregnated with, but to a degree which would not allow a leakage of, the cosmetic preparation composition of Examples 13 and 13A to 13D, or Comparative Examples 8 and 9, while loading a pressure of about 200 g in a reciprocating manner thirty times. From the proportion of the line painted with the oil paint pen which had been able to be removed by this removal operation, the cleansing properties of the respective cosmetic preparation compositions as an ink remover ingredient were evaluated in accordance with the following criteria. The results of their averages are shown in Table 14.

### <Evaluation criteria of cleansing properties>

Excellent: High cleansing properties, meaning that a proportion of 75 to 100% in the line painted with the oil paint pen was able to be removed.
Good: Relatively high cleansing properties, meaning that a proportion of 50 to 75% in the line painted with the oil paint pen was able to be removed.
Moderate: Some cleansing properties, meaning that only a proportion of 25 to 50% in the line painted with the oil paint pen was able to be removed.
Poor: Weak cleansing properties, meaning that only a proportion of 0 to 25% in the line painted with the oil paint pen was able to be removed.

Moreover, the low irritation properties of the respective cosmetic preparation compositions as an ink remover ingredient were evaluated in the following manner.
The insides of the arms of seven panelists were washed with soap and water, and in addition, the washed surfaces were wiped with ethanol and naturally dried. Next, each of the thus washed surfaces was evenly divided into seven equal areas, to which 0.01 g of the respective cosmetic preparation compositions of Examples 13 and 13A to 13D, and Comparative Examples 8 and 9, was applied airtightly by using Finn Chamber (a product of Epitest Ltd Oy). After 24 hours, the Finn Chamber and the cosmetic preparation compositions were taken off. After 1 hour, the condition of the skin was observed. The results of their averages are shown in Table 14.
The low irritation properties of the respective cosmetic preparation compositions as an ink remover ingredient were evaluated in accordance with the following criteria.

### <Evaluation criteria of low irritation properties>

Good: No change was found in the skin of the airtightly applied part.
Moderate: A slight rash was found in the skin of the airtightly applied part.
Poor: A notable rash was found in the skin of the airtightly applied part.

**[Table 14]**

| Cosmetic preparation composition to remove ink of oil paint pen | | | | | | | |
|---|---|---|---|---|---|---|---|
| | Comparative Example 8 | Comparative Example 9 | Example 13 | Example 13A | Example 13B | Example 13C | Example 13D |
| Cleansing properties | Good | Moderate | Excellent | Excellent | Good | Moderate | Poor |
| Low irritation properties | Poor | Good | Good | Moderate | Good | Good | Good |

As a result, not much change was found in the skin of the part airtightly applied with the cosmetic preparation compositions of Examples 13 and 13A to 13D, and Comparative Example 9, whereas a notable rash was found in the skin of the part airtightly applied with the cosmetic preparation composition of Comparative Example 8.

From the above-mentioned results, it is apparent that the cosmetic preparation compositions of Examples 13 and 13A to 13D according to the present invention have equivalent or better cleansing properties and/or low irritation properties than those of the cosmetic preparation compositions of Comparative Examples 8 and 9, when used as an ingredient of an ink of an oil paint pen for the skin.

### [Examples 14, 14A, and 14B, and Comparative Examples 10, 11, 11A, and 11B]

In Example 14, the PDO-C8/C10 diester obtained from Synthesis Example 2 was used as an industrial use cleanser (agent to remove an oil soluble colorant) for a coated steel panel, and the cleansing properties and the low corrosion properties thereof were evaluated.
In Examples 14A and 14B, the PDO-C8/C10 = 50:50 diester obtained from Synthesis Example 7, or the PDO-C8/C10 = 25:75 diester obtained from Synthesis Example 8, were respectively used instead of the PDO-C8/C10 diester obtained from Synthesis Example 2, as an industrial use cleanser (agent to remove an oil soluble colorant) for a coated steel panel, and the cleansing properties and the low corrosion properties thereof were evaluated in the same manners as those of Example 14.
Regarding Comparative Examples 10 and 11 and Comparative Examples 11A and 11B, fossil fuel-derived ethanol (a product of Wako Pure Chemical Industries, Ltd.) was used in Comparative Example 10, a fossil fuel-derived mineral spirit A (a product of Nippon Oil Corporation) was used in Comparative Example 11, the PDO-C8 diester obtained from Synthesis Example 6 was used in Comparative Example 11A, and the PDO-C10 diester obtained from Synthesis Example 9 was used in Comparative Example 11B.

The cleansing properties of the respective industrial use cleansers as an agent to remove an oil soluble colorant from a coated steel panel were evaluated in the following manner.
First, an electro-galvanized steel panel was coated with commercially available automotive paints, namely, a primer surfacer (Product Name: Primer White (a product of Musashi Holt Co., Ltd.)), a white automotive paint (Product Name: Antirust Paint (Color No. 041) (a product of Musashi Holt Co., Ltd.)), and a clear paint (Product Name: Topcoat Clear (a product of Musashi Holt Co., Ltd.)), by spraying according to the usage instruction of each product, and dried at 25°C for 24 hours. By so doing, a coated steel panel for the test was obtained.
Next, using a black oil paint pen (Product Name: Mckee Care, a product of Zebra Co., Ltd.), a line was painted on the surface of the coated steel panel, and dried at 25°C for 24 hours.
Subsequently, the painted line was evenly divided into seven equal areas. Each area was rubbed with an absorbent cotton that had been impregnated with, but to a degree which would not allow a leakage of, the industrial use cleanser of Examples 14, 14A, and 14B, or the oil agent of Comparative Examples 10, 11, 11A, and 11B, while loading a pressure of about 200 g in a reciprocating manner thirty times. From the proportion of the ink of the line painted with the oil paint pen which had been able to be removed by this removal operation, the cleansing properties of the respective industrial use cleansers of Examples 14, 14A, and 14B, or the respective oil agents of Comparative Examples 10, 11, 11A, and 11B, as the agent to remove the oil soluble colorant, were evaluated by eye in accordance with the following criteria. The results are shown in Table 15.

### <Evaluation criteria of cleansing properties>

Excellent: High cleansing properties, meaning that a proportion of 75 to 100% in the line painted with the oil paint pen on the coated steel panel was able to be removed.
Good: Relatively high cleansing properties, meaning that a proportion of 50 to 75% in the line painted with the oil paint pen on the coated steel panel was able to be removed.
Moderate: Some cleansing properties, meaning that only a proportion of 25 to 50% in the line painted with the oil paint pen on the coated steel panel was able to be removed.
Poor: Weak cleansing properties, meaning that only a proportion of 0 to 25% in the line painted with the oil paint pen on the coated steel panel was able to be removed.

Next, the corrosion properties of the industrial use cleanser of Examples 14, 14A, and 14B, or the oil agent of Comparative Examples 10, 11, 11A, and 11B, for the coated steel panel, were evaluated in the following manner.
An absorbent cotton impregnated with, but to a degree which would not allow a leakage of, the industrial use cleanser of Examples 14, 14A, and 14B, or the oil agent of Comparative Examples 10, 11, 11A, and 11B, was left still on the coated surface of the coated steel panel for the test for 6 hours. Thereafter, the absorbent cotton was taken off, and the corrosion of the coated surface was evaluated by eye in accordance with the following criteria.
The results are shown in Table 15.

### <Evaluation criteria of low corrosion properties>

Good: No change was found in the coated surface.
Moderate: The paint of the coated surface was slightly corroded, and the coated surface was slightly rugged.
Poor: The paint of the coated surface was remarkably corroded, and the coated surface was rugged.

**[Table 15]**

| Industrial use cleanser | | | | | | | |
|---|---|---|---|---|---|---|---|
| | Comparative Example 10 | Comparative Example 11 | Comparative Example 11A | Comparative Example 11B | Example 14 | Example 14A | Example 14B |
| Cleansing properties | Excellent | Excellent | Excellent | Poor | Excellent | Good | Moderate |
| Low corrosion properties | Poor | Moderate | Moderate | Good | Good | Good | Good |

From the above-mentioned results, it is apparent that the industrial use cleansers of Examples 14, 14A, and 14B according to the present invention have equivalent or better cleansing properties and/or low corrosion properties than those of the oil agents of Comparative Examples 10, 11, 11A, and 11B, when used as an agent to remove an ink of an oil paint pen for a coated steel panel. Moreover, since the industrial use cleansers of Examples 14, 14A, and 14B are all derived from plant or microbial fermentation, the load on the environment is reduced as compared to the cases of the oil agents of Comparative Examples 10 and 11.

### [Examples 15 and 15A to 15C, and Comparative Examples 12, 13, and 12A to 13B]

An industrial use cleanser including the PDO-C8/C10 diester obtained from Synthesis Example 2 was used as an agent to remove an oil soluble colorant (agent to remove a marker ink) for a coated steel panel, and the cleansing properties and the low corrosion properties thereof were evaluated.

In Examples 15 and 15A to 15C, the PDO-C8/C10 diester obtained from Synthesis Example 2, purified water, a microbial fermentation-derived xanthan gum (Product Name: Nomu-coat ZZ, a product of the Nisshin OilliO Group, Ltd.), and palm-derived or palm kernel-derived polyglyceryl laurate (the average degree of polymerization of polyglycerin was 10, and the saponification value was from 35 to 75) (Product Name: Sun Soft Q-12S, a product of Taiyo Kagaku Co., Ltd.) were respectively mixed in accordance with the parts by mass as shown in Table 16 and Table 17, thereby preparing ingredients A to C.
Moreover, in Comparative Examples 12 and 12A to 13B, a fossil fuel-derived ethanol (a product of Wako Pure Chemical Industries, Ltd.), or a fossil fuel-derived mineral spirit A (a product of Nippon Oil Corporation), were respectively used instead of the above-mentioned PDO-C8/C10 diester.
The amount of CO₂ (kg) emitted to the environment upon burning of 1t of an industrial use cleanser was calculated in the same manner as that of Example 1 to calculate the amount of CO₂ (kg) emitted to the environment upon burning of 1t of a cosmetic preparation.

Next, the ingredient B that had been heated and melted at 80°C was charged into a homomixer (Model Name: T. K. Agihomomixer 2M-05 type, a product of Primix Corporation), to which the ingredient A that had been heated at 60°C was gradually added under stirring. Thereafter, the ingredient C was added thereto, and the mixture was cooled down to room temperature. By so doing, industrial use cleansers in forms of milky white liquid were obtained.

The cleansing properties and the low corrosion properties of the respective industrial use cleansers as an agent to remove an oil soluble colorant for a coated steel panel were evaluated in the same manner as those of the industrial use cleanser in Example 14.
The results are shown in Table 16 and Table 17.

**[Table 16] Industrial use cleanser (agent to remove a marker ink)**

| Ingredients | | Comparative Example 12 | Comparative Example 13 | Example 15 |
|---|---|---|---|---|
| A | PDO-C8/C10 diester | 0 | 0 | 35.0 |
| | Ethanol | 35.0 | 0 | 0 |
| | Mineral spirit A | 0 | 35.0 | 0 |
| B | Purified water | 62.8 | 62.8 | 62.8 |
| | Polyglyceryl-10 laurate | 2.0 | 2.0 | 2.0 |
| C | Xanthan gum | 0.2 | 0.2 | 0.2 |
| Amount of CO₂ (kg) emitted to the environment upon burning of It | | 67 | 87 | 0 |
| Cleansing properties | | Excellent | Excellent | Excellent |
| Low corrosion properties | | Poor | Moderate | Good |

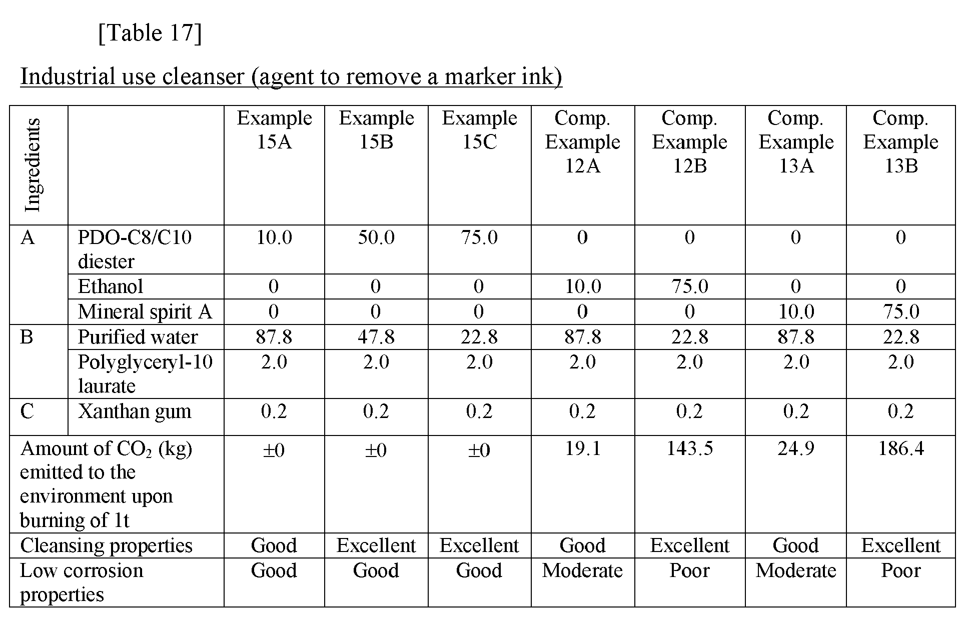

From the above-mentioned results, it is apparent that the industrial use cleansers of Examples 15 and 15A to 15C according to the present invention have equivalent cleansing properties to those of the industrial use cleansers of Comparative Examples 12, 13, and 12A to 13B, as well as having better low corrosion properties than those of the industrial use cleansers of Comparative Examples 12 and 13 when used as an agent to remove an oil soluble colorant for a coated steel panel. Moreover, since all the raw materials (except for purified water) of the industrial use cleansers of Examples 15 and 15A to 15C are derived from plant or microbial fermentation, the load on the environment is reduced as compared to the cases of the industrial use cleansers of Comparative Examples 12, 13, and 12A to 13B.

### [Examples 16, 16A, and 16B, and Comparative Examples 14, 15, and 14A to 15C]

An industrial use cleanser including the PDO-C8/C10 diester obtained from Synthesis Example 2 was used as an agent to remove an oil soluble colorant (agent to remove a marker ink) for a coated steel panel, and the cleansing properties and the low corrosion properties thereof were evaluated.

In Examples 16, 16A, and 16B, the PDO-C8/C10 diester obtained from Synthesis Example 2 and a nitrogen gas were filled in accordance with the parts by mass as shown in Table 18 and Table 19, into an aerosol can.
Moreover, in Comparative Examples 14, 15, and 14A to 15C, a fossil fuel-derived ethanol (a product of Wako Pure Chemical Industries, Ltd.), or a fossil fuel-derived mineral spirit A (a product of Nippon Oil Corporation), were respectively used instead of the above-mentioned PDO-C8/C10 diester.
The amount of CO₂ (kg) emitted to the environment upon burning of 1t of an industrial use cleanser was calculated in the same manner as that of Example 1 to calculate the amount of CO₂ (kg) emitted to the environment upon burning of 1t of a cosmetic preparation.

The cleansing properties of the respective industrial use cleansers as an agent to remove an oil soluble colorant from a coated steel panel was evaluated in the following manner.
Using a black oil paint pen (Product Name: Mckee Care, a product of Zebra Co., Ltd.), a line was painted on the surface of the coated steel panel for the test, and dried at 25°C for 24 hours.
Next, an absorbent cotton was impregnated with, but to a degree which would not allow a leakage of, the industrial use cleanser by spraying from the aerosol can that had been filled with the cleanser. Subsequently, the painted line was evenly divided into ten equal areas. Each area was rubbed with an absorbent cotton that had been impregnated with, but to a degree which would not allow a leakage of, the industrial use cleanser of Examples 16, 16A, and 16B, or the industrial use cleanser of Comparative Examples 14, 15, and 14A to 15C, while loading a pressure of about 200 g in a reciprocating manner thirty times. From the proportion of the ink of the line painted with the oil paint pen which had been able to be removed by this removal operation, the cleansing properties of the respective industrial use cleansers as the agent to remove the oil soluble colorant were evaluated in accordance with the same criteria as those of the industrial use cleanser in Example 15 mentioned above. The results are shown in Table 18 and Table 19.

The corrosion properties of the respective industrial use cleansers for the coated steel panel were evaluated in the following manner.
An absorbent cotton was impregnated with, but to a degree which would not allow a leakage of, the industrial use cleanser of Examples 16, 16A, and 16B, or Comparative Examples 14, 15, and 14A to 15C, by spraying from the aerosol can that had been filled with the cleanser. The absorbent cotton impregnated with each industrial use cleanser was left still on the coated surface of the coated steel panel for the test for 6 hours. Thereafter, the absorbent cotton was taken off, and the corrosion of the coated surface was evaluated in accordance with the same criteria as those of the industrial use cleanser in Example 15 mentioned above.
The results are shown in Table 18 and Table 19.

**[Table 18]**

| Industrial use cleanser (aerosol) | | | |
|---|---|---|---|
| | Comparative Example 14 | Comparative Example 15 | Example 16 |
| PDO-C8/C10 diester | 0 | 0 | 90.0 |
| Ethanol | 90.0 | 0 | 0 |
| Mineral spirit A | 0 | 90.0 | 0 |
| Nitrogen (Spray gas agent) | 10.0 | 10.0 | 10.0 |
| Amount of CO₂ (kg) emitted to the environment upon burning of 1t | 172.2 | 223.7 | ±0 |
| Cleansing properties | Excellent | Excellent | Excellent |
| Low corrosion properties | Poor | Moderate | Good |

**[Table 19]**

| Industrial use cleanser (aerosol) | | | | | | | |
|---|---|---|---|---|---|---|---|
| | Example 16A | Example 16B | Comp. Example 14A | Comp. Example 14B | Comp. Example 15A | Comp. Example 15B | Comp. Example 15C |
| PDO-C8/C10 diester | 5.0 | 50.0 | 0 | 0 | 0 | 0 | 0 |
| Ethanol | 0 | 0 | 5.0 | 50.0 | 0 | 0 | 0 |
| Mineral spirit A | 0 | 0 | 0 | 0 | 5.0 | 50.0 | 0 |
| Soybean oil YM | 85.0 | 40.0 | 85.0 | 40.0 | 85.0 | 40.0 | 90.0 |
| Nitrogen (Spray gas agent) | 10.0 | 10.0 | 10.0 | 10.0 | 10.0 | 10.0 | 10.0 |
| Amount of CO₂ (kg) emitted to the environment upon burning of 1t | ±0 | ±0 | 9.6 | 95.7 | 12.4 | 124.3 | ±0 |
| Cleansing properties | Good | Excellent | Good | Excellent | Good | Excellent | Poor |
| Low corrosion properties | Good | Good | Moderate | Poor | Moderate | Moderate | Good |

From the above-mentioned results, it is apparent that the industrial use cleansers of Examples 16, 16A, and 16B according to the present invention have equivalent cleansing properties to those of the industrial use cleansers of Comparative Examples 14, 15, and 14A to 15C, as well as having better low corrosion properties than those of the industrial use cleansers of Comparative Examples 14, 15, and 14A to 15C when used as an agent to remove an oil soluble colorant for a coated steel panel. Moreover, since all the raw materials (except for nitrogen) of the industrial use cleansers of Examples 16, 16A, and 16B are derived from plant or microbial fermentation, the load on the environment is reduced as compared to the cases of the industrial use cleansers of Comparative Examples 14, 15, and 14A to 15C.

### [Examples 17 to 21 and Comparative Examples 16 to 18]

In Examples 17 to 21, the PDO diisostearate obtained from Synthesis Example 1, hydrogenated soybean-derived lecithin (Product Name: Basis LS-60HR, a product of the Nisshin OilliO Group, Ltd.), palm-derived glycerin (a product of Sakamoto Yakuhin Kogyo Co., Ltd.), microbial fermentation-derived 1,3-propanediol (Product Name: Zemea Propanediol, a product of DuPont Tate & Lyle BioProducts), and purified water were respectively mixed in accordance with the parts by mass as shown in Table 20, thereby preparing an ingredient A and an ingredient B.
Moreover, in Comparative Examples 16 to 18, the PDO multibranched diisostearate obtained from Synthesis Example 3, the PDO-Garbett isostearic acid diester obtained from Synthesis Example 4, or fossil fuel-derived hydrogenated polydecene (Product Name: Nomu-coat HP-100 (a product of the Nisshin OilliO Group, Ltd.), were respectively used instead of the above-mentioned PDO diisostearate.

Next, the ingredient B that had been heated and melted at 80°C was charged into a homodisper (Product Name: T. K. Agihomomixer 2M-05 type, a product of Primix Corporation), to which the ingredient A that had been heated at 80°C was gradually added under stirring by means of the rotation of a disper blade at 3000 rpm. On completion of the addition, the mixture was kept stirred for 10 minutes and cooled down to 55°C. The rotation of the disper blade was stopped, and the mixture was further cooled down to room temperature. By so doing, massage gels were obtained.

The moisturizing properties and the feeling of use of the obtained massage gels were evaluated in the following manners.
The insides of the arms of seven panelists were washed with soap and water, and in addition, the washed surfaces were wiped with ethanol and naturally dried. Thereafter, each of the thus washed surfaces was evenly divided into eight equal areas, to which 0.05 g of the respective massage gel of Examples 17 to 21, and Comparative Examples 16 to 18, was applied. The area was gently rubbed with a finger ten times, and then wiped with a dry cloth. On completion of this wiping, the moisturizing properties and the feeling of use of the respective massage gel were evaluated in accordance with the following criteria. The results of their averages are shown in Table 20.

### <Evaluation criteria of moisturizing properties>

Good: Very soft and moistened touch was felt.
Moderate: Soft and moistened touch was felt.
Poor: No moistened touch was felt.

### <Evaluation criteria of feeling of use>

Good: No sticky texture was felt.
Moderate: Slightly sticky texture was felt.
Poor: Sticky texture was felt.

### <Amount of CO₂ (kg) emitted to the environment upon burning of 1t of cosmetic preparation>

The amount of CO₂ emitted to the environment upon burning of 1t of the cosmetic preparation was calculated based on the fossil fuel-derived raw material in accordance with the following equation (in view of the carbon neutral concept, the amount of CO₂ emitted from plant-derived and microorganism-derived raw materials was deemed to be ±0).
Amount of CO₂ (kg) emitted to the environment upon burning of 1t of cosmetic preparation = (Quantity of mixed fossil fuel-derived raw material/Molecular weight of the raw material) × Carbon number of the raw material × 44 (Molecular weight of carbon dioxide)

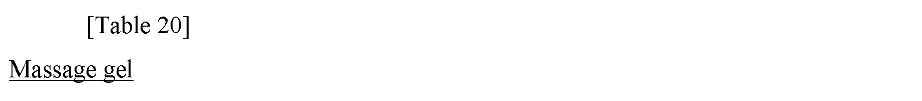

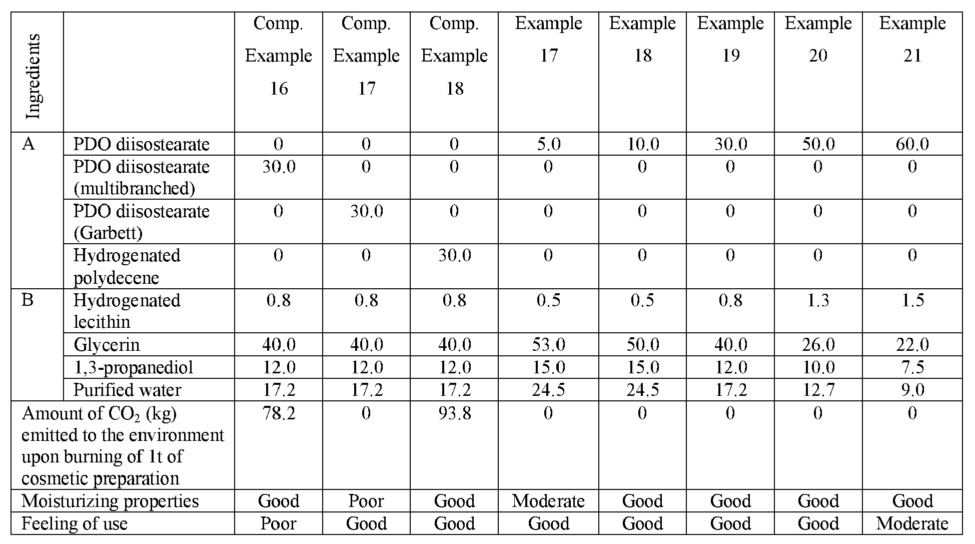

From the above-mentioned results, it is apparent that the massage gels of Examples 17 to 21 according to the present invention have equivalent or better moisturizing properties and/or feeling of use than those of the massage gels of Comparative Examples 16 to 18. Moreover, since all the raw materials (except for purified water) of the massage gels of Examples 17 to 21 are derived from plant or microbial fermentation, the load on the environment is reduced as compared to the cases of the massage gels of Comparative Examples 16 and 18, as apparent from the amount of CO₂ emitted to the environment (kg) upon burning of 1t.

### [Examples 22 to 26 and Comparative Examples 19 to 21]

In Examples 22 to 26, the PDO diisostearate obtained from Synthesis Example 1, glyceryl behenate/eicosadioate (Product Name: Nomu-coat HK-G, a product of the Nisshin OilliO Group, Ltd.), polyglyceryl-10 behenate/eicosadioate (Product Name: Nomu-coat HK-P, a product of the Nisshin OilliO Group, Ltd.), palm-derived glycerin (a product of Sakamoto Yakuhin Kogyo Co., Ltd.), microbial fermentation-derived 1,3-propanediol (Product Name: Zemea Propanediol, a product of DuPont Tate & Lyle BioProducts), diglycerin, and purified water were respectively mixed in accordance with the parts by mass as shown in Table 21, thereby preparing an ingredient A and an ingredient B.
Moreover, in Comparative Examples 19 and 20, the PDO multibranched diisostearate obtained from Synthesis Example 3, the PDO-Garbett isostearic acid diester obtained from Synthesis Example 4, or fossil fuel-derived hydrogenated polydecene (Product Name: Nomu-coat HP-100, a product of the Nisshin OilliO Group, Ltd.), were respectively used instead of the above-mentioned PDO diisostearate.

Next, the ingredient B that had been heated and melted at 80°C was charged into a homodisper (Product Name: T. K. Agihomomixer 2M-05 type, a product of Primix Corporation), to which the ingredient A that had been heated at 80°C was gradually added under stirring by means of the rotation of a disper blade at 2000 rpm. On completion of the addition, the rotation speed of the disper blade of the stirrer was slowed down to 1500 rpm. The mixture was cooled down to room temperature. By so doing, moisturizing lip balms were obtained.

The moisturizing properties, the feeling of use, and the stability of the obtained moisturizing lip balms were evaluated for in the following manners.
The lips of seven panelists were washed with soap and water, and then naturally dried. Thereafter, 0.05 g of each type of the moisturizing lip balms of Examples 22 to 26 and Comparative Examples 19 to 21 was applied and gently rubbed with a finger ten times. On completion of this application, the moisturizing properties and the feeling of use of the respective moisturizing lip balm were evaluated in accordance with the following criteria. All the evaluations of Examples and Comparative Examples were conducted with one hour intervals. The results of their averages are shown in Table 21.

### <Evaluation criteria of moisturizing properties>

Good: Very soft and moistened touch was felt.
Moderate: Soft and moistened touch was felt.
Poor: No moistened touch was felt.

### <Evaluation criteria of feeling of use>

Good: No sticky texture was felt.
Moderate: Slightly sticky texture was felt.
Poor: Sticky texture was felt.

### <Evaluation criteria of stability>

Good: No odor was noticed after a month storage at 50°C.
Poor: An odor was noticed after a month storage at 50°C.

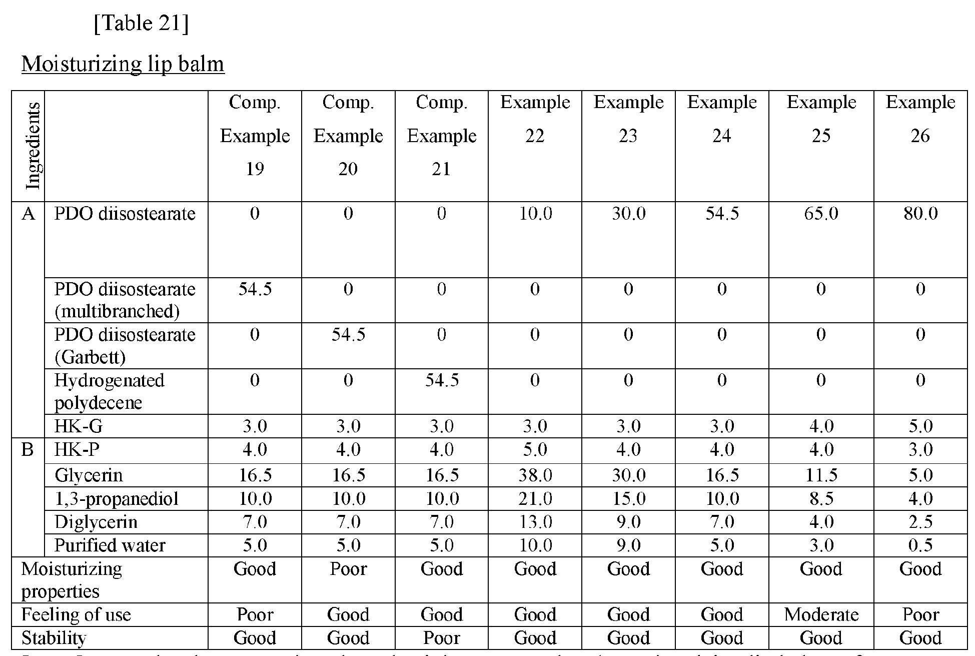

From the above-mentioned results, it is apparent that the moisturizing lip balms of Examples 22 to 26 according to the present invention have equivalent or better moisturizing properties, feeling of use, and/or stability than those of the moisturizing lip balms of Comparative Examples 19 to 21.

### [Example 27 and Comparative Examples 22 to 27]

In Example 27, the PDO-C8/C10 diester obtained from Synthesis Example 2, hydrogenated soybean-derived lecithin (Product Name: Basis LS-60HR, a product of the Nisshin OilliO Group, Ltd.), palm-derived glycerin (a product of Sakamoto Yakuhin Kogyo Co., Ltd.), microbial fermentation-derived 1,3-propanediol (Product Name: Zemea Propanediol, a product of DuPont Tate & Lyle BioProducts), purified water, and a quince seed powder (Product Name: Quince Seed Powder, a product of Taiyo Kagaku Co., Ltd.) were respectively mixed in accordance with the parts by mass as shown in Table 22, thereby preparing an ingredient A, an ingredient B, and an ingredient C.
Moreover, in Comparative Examples 22 to 27, fossil fuel-derived isononyl isononanoate (Product Name: Salacos 99, a product of the Nisshin OilliO Group, Ltd.) or any one of the following mineral-originated silicone oils (*products of Shin-Etsu Chemical Co., Ltd.), were respectively used instead of the above-mentioned PDO-C8/C10 diester.
* Decamethyl cyclopentasiloxane (Product Name: KF-995);
Methyl polysiloxane 5 cs (Product Name: KF-96A-5cs);
Methyl polysiloxane 10 cs (Product Name: KF-96A-10cs);
Methyl polysiloxane 50 cs (Product Name: KF-96A-50cs);
Methyl polysiloxane 100 cs (Product Name: KF-96A-100cs)

Next, the ingredient B that had been heated and melted at 80°C was charged into a homodisper (Product Name: T. K. Agihomomixer 2M-05 type, a product of Primix Corporation), to which the ingredient A that had been heated at 80°C was gradually added under stirring by means of the rotation of a disper blade at 5000 rpm. On completion of the addition, the rotation speed of the disper blade of the stirrer was slowed down to 1500 rpm. At a point of time when the mixture was cooled down to 40°C, the ingredient C was added. The mixture was cooled down to room temperature. By so doing, moisturizing emulsions in forms of milky white turbid liquid (moisturizing cosmetic preparations) were obtained.
The feeling of use of the obtained moisturizing emulsions during the application, the stickiness thereof after 30 minutes from the application (property to fit the skin), and also the storage stability and the low irritation properties of these cosmetic preparations, were evaluated in the following manners.
The insides of arms of ten panelists were washed with soap and water, and then naturally dried. Thereafter, 0.1 g of each type of respective moisturizing emulsions of the Example 27 and Comparative Examples 22 to 27 was applied and gently rubbed with a finger ten times. The feeling of use of the moisturizing emulsions during the application was evaluated in accordance with the following criteria. In addition, the stickiness and the low irritation properties of the applied part after 30 minutes from the application were evaluated in accordance with the following criteria. All the evaluations of Examples and Comparative Examples were conducted with one hour intervals. Moreover, the storage stability of the cosmetic preparation was checked by eye by watching the condition of the appearance of the prepared cosmetic preparation after leaving still in a 50°C thermostat for 2 weeks.
The results of their averages are shown in Table 22.

### <Evaluation criteria of smoothness during application>

Excellent: Very light touch, spread well, and very smooth during the application.
Good: Very light touch, but rough friction was felt during the application.
Moderate: Heavy touch and spread unwell during the application.

### <Evaluation criteria of stickiness after 30 minutes from application (property to fit the skin)>

Excellent: No sticky texture was felt.
Good: Slightly sticky texture was felt.
Moderate: Notably sticky texture was felt.

### <Evaluation criteria of storage stability of cosmetic preparation>

Good: No conditional change was found after the accelerated test of storage stability for two weeks at 50°C.
Moderate: Low syneresis of oil and water was found after the accelerated test of storage stability for two weeks at 50°C.
Poor: The emulsification system was ruptured and a phase separation was found after the accelerated test of storage stability for two weeks at 50°C.

### <Evaluation criteria of low irritation properties>

A: Almost no tingling irritation was felt.
B: Tingling irritation was felt.

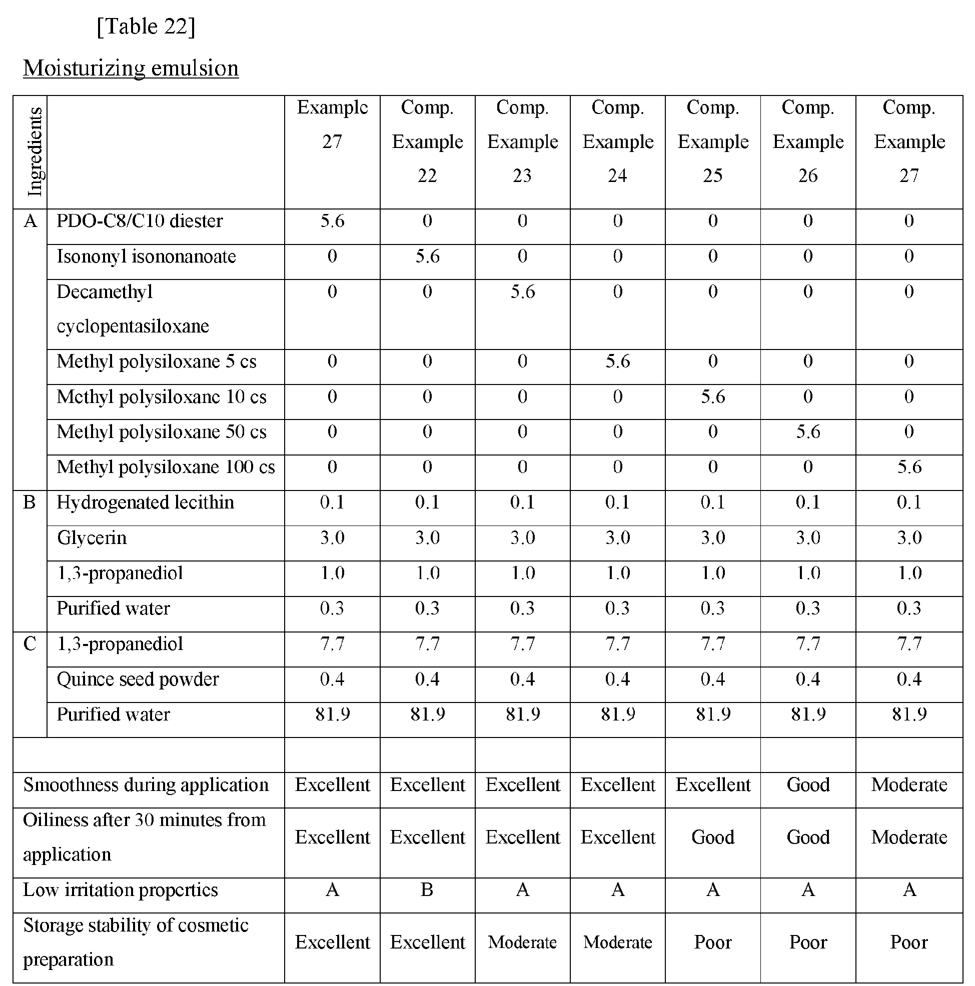

From the above-mentioned results, it is apparent that the moisturizing emulsion of Example 27 according to the present invention has equivalent or better feeling of use and/or low irritation properties than those of the moisturizing emulsions of Comparative Examples 22 to 27, as well as having excellent stability.

[Examples 28 and 29 and Comparative Examples 28 to 32] In Example 28, the PDO-C8/C10 diester obtained from Synthesis Example 2, isostearic acid trehalose esters comprising microbial fermentation-derived trehalose and plant-derived isostearic acid (Product Name: Nomu-coat TQ-5, a product of the Nisshin OilliO Group, Ltd.) as an emulsifying agent, microbial fermentation-derived 1,3-propanediol (Product Name: Zemea Propanediol, a product of DuPont Tate & Lyle BioProducts) as a water phase ingredient, purified water, and sodium chloride (a product of Wako Pure Chemical Industries, Ltd.) as an emulsion stabilizer were mixed in accordance with the parts by mass as shown in Table 23, thereby preparing an ingredient A and an ingredient B.
Moreover, in Comparative Examples 28 to 30, decamethyl cyclopentasiloxane (Product Name: KF-995, a product of Shin-Etsu Chemical Co., Ltd.), methyl polysiloxane 10 cs (Product Name: KF-96A-10cs, a product of Shin-Etsu Chemical Co., Ltd.) or methyl polysiloxane 100 cs (Product Name: KF-96A-100cs, a product of Shin-Etsu Chemical Co., Ltd.), were respectively used instead of the above-mentioned PDO-C8/C10 diester, and cetyl dimethicone copolyol (Product Name: ABIL EM-90, a product of Evonic Industries) was used instead of the isostearic acid trehalose esters, as an emulsifying agent, to prepare the ingredient A.
In Example 29, a part of the PDO-C8/C10 diester was replaced by the PDO diisostearate obtained from Synthesis Example 1, to prepare the ingredient A.
In Comparative Examples 31 and 32, a part of the decamethyl cyclopentasiloxane was replaced by methyl polysiloxane (Product Name: KF-96A-10 cs and KF-96A-100 cs, products of Shin-Etsu Chemical Co., Ltd.), to prepare the ingredient A.

Next, the ingredient A that had been heated and melted at 80°C was charged into a homomixer (Product Name: T. K. Agihomomixer 2M-05 type, a product of Primix Corporation), to which the ingredient B that had been heated at 80°C was gradually added under stirring by means of the rotation of a mixer turbine at 7000 rpm. On completion of the addition, the rotation speed of the turbine of the mixer was slowed down to 5000 rpm. The mixture was cooled down to room temperature. By so doing, moisturizing creams were obtained.
The feeling of use of the obtained moisturizing creams during the application, the stickiness thereof after 30 minutes from the application (property to fit the skin), and the low irritation properties thereof were evaluated in the following manners.
The insides of arms of ten panelists were washed with soap and water, and then naturally dried. Thereafter, 0.05 g of each type of the moisturizing creams of Examples 28 and 29 and Comparative Examples 28 to 32 was applied and gently rubbed with a finger ten times. The feeling of use of the moisturizing creams during the application was evaluated in accordance with the following criteria. In addition, the stickiness (property to fit the skin) and the low irritation properties of the applied part after 30 minutes from the application were also evaluated in accordance with the following criteria. All the evaluations of Examples and Comparative Examples were conducted with one hour intervals.
The results of their averages are shown in Table 23.

### <Evaluation criteria of smoothness during application>

Excellent: Very light touch, spread well, and very smooth during the application.
Good: Very light touch, but rough friction was felt during the application.

### <Evaluation criteria of stickiness after 30 minutes from application (property to fit the skin)>

Excellent: No sticky texture was felt.
Good: Slightly sticky texture was felt.

### <Evaluation criteria of low irritation properties>

A: Almost no tingling irritation was felt.
B: Tingling irritation was felt.

**[Table 23]**

| Moisturizing cream | | | | | | | | |
|---|---|---|---|---|---|---|---|---|
| | | Example 28 | Example 29 | Comp. Example 28 | Comp. Example 29 | Comp. Example 30 | Comp. Example 31 | Comp. Example 32 |
| A Oil phase/ emulsifying agent | PDO-C8/C10 diester | 44.0 | 40.0 | 0 | 0 | 0 | 0 | 0 |
| | PDO diisostearate | 0 | 4.0 | 0 | 0 | 0 | 0 | 0 |
| | Decamethyl cyclopentasiloxane | 0 | 0 | 44.0 | 0 | 0 | 30.0 | 30.0 |
| | Methyl polysiloxane 10 cs | 0 | 0 | 0 | 44.0 | 0 | 14.0 | 10.0 |
| | Methyl polysiloxane 100 cs | 0 | 0 | 0 | 0 | 44.0 | 0 | 4.0 |
| | Cetyl dimethicone copolyol | 0 | 0 | 5.0 | 5.0 | 5.0 | 5.0 | 5.0 |
| | Isostearic acid trehalose esters | 5.0 | 5.0 | 0 | 0 | 0 | 0 | 0 |
| B Water phase | 1,3-propanediol | 5.0 | 5.0 | 5.0 | 5.0 | 5.0 | 5.0 | 5.0 |
| | Sodium chloride | 1.0 | 1.0 | 1.0 | 1.0 | 1.0 | 1.0 | 1.0 |
| | Purified water | 45.0 | 45.0 | 45.0 | 45.0 | 45.0 | 45.0 | 45.0 |
| | | | | | | | | |
| Smoothness during application | | Excellent | Good | Excellent | Excellent | Good | Excellent | Good |
| Oiliness after 30 minutes from application | | Excellent | Good | Excellent | Excellent | Good | Excellent | Good |
| Low irritation properties | | A | A | A | A | A | A | A |

From the above-mentioned results, it is apparent that the moisturizing creams of Example 28 and 29 according to the present invention have equivalent or better feeling of use and/or low irritation properties than those of the moisturizing creams of Comparative Examples 28 to 32.

### INDUSTRIAL APPLICABILITY

The present invention is useful in the fields of cosmetic preparations or industrial use cleansing agents.

## Claims

1. A cosmetic preparation comprising an emulsifying agent, 1,3-propanediol, and a 1,3-propanediol difatty acid ester, wherein the fatty acids constituting said 1,3-propanediol difatty acid ester are one or two or more types of fatty acids selected from isostearic acid, n-octanoic acid, and n-decanoic acid, and said isostearic acid has a structure in which one or two methyl groups are branched from the principal chain of stearic acid.

2. The cosmetic preparation according to claim 1, wherein the mass ratio of said 1,3-propanediol to said 1,3-propanediol difatty acid ester is within a range of 3:1 to 1:8.

3. The cosmetic preparation according to claim 1 or 2, wherein the fatty acids constituting said 1,3-propanediol difatty acid ester are n-octanoic acid and n-decanoic acid.

4. The cosmetic preparation according to claim 3, wherein the fatty acids constituting said 1,3-propanediol difatty acid ester are n-octanoic acid and n-decanoic acid, and said 1,3-propanediol difatty acid ester is contained in a proportion of 5.0 to 60.0% by mass.

5. The cosmetic preparation according to claim 4, wherein the fatty acids constituting said 1,3-propanediol difatty acid ester are n-octanoic acid and n-decanoic acid, said 1,3-propanediol difatty acid ester is contained in a proportion of 5.0 to 60.0% by mass, said emulsifying agent is contained in a proportion of 0.05 to 10.0% by mass, and said 1,3-propanediol is contained in a proportion of 3.0 to 50.0% by mass.

6. The cosmetic preparation according to claim 3, wherein the mass ratio of the n-octanoic acid and the n-decanoic acid which constitute said 1,3-propanediol difatty acid ester is from 95:5 to 5:95.

7. The cosmetic preparation according to claim 3, wherein said cosmetic preparation is a cleansing cosmetic preparation, and the mass ratio of the n-octanoic acid and the n-decanoic acid which constitute said 1,3-propanediol difatty acid ester is from 90:10 to 40:60.

8. The cosmetic preparation according to claim 3, wherein said cosmetic preparation is a cleansing cosmetic preparation, and the mass ratio of the n-octanoic acid and the n-decanoic acid which constitute said 1,3-propanediol difatty acid ester is from 75:25 to 25:75.

9. The cosmetic preparation according to claim 1 or 2, wherein the fatty acids constituting said 1,3-propanediol difatty acid ester are isostearic acids.

10. The cosmetic preparation according to claim 9, wherein said isostearic acid has the following properties: the viscosity with a shear of 10 Pa is from 99 to 130 mPa·s at 10°C, and the isostearic acid is in a liquid state at 5°C.

11. The cosmetic preparation according to claim 9 or 10, wherein the fatty acids constituting said 1,3-propanediol difatty acid ester are isostearic acids, and said 1,3-propanediol difatty acid ester is contained in a proportion of 2.0 to 75.0% by mass.

12. The cosmetic preparation according to claim 11, wherein the fatty acids constituting said 1,3-propanediol difatty acid ester are isostearic acids, said 1,3-propanediol difatty acid ester is contained in a proportion of 2.0 to 75.0% by mass, said emulsifying agent is contained in a proportion of 0.05 to 10.0% by mass, and said 1,3-propanediol is contained in a proportion of 7.0 to 25.0% by mass.

13. The cosmetic preparation according to any one of claims 1 to 6 and claims 9 to 12, wherein said cosmetic preparation is a cleansing cosmetic preparation.

14. The cosmetic preparation according to claim 3 or 6, wherein said cosmetic preparation is a cleansing cosmetic preparation, the fatty acids constituting said 1,3-propanediol difatty acid ester are n-octanoic acid and n-decanoic acid, said 1,3-propanediol difatty acid ester is contained in a proportion of 40.0 to 60.0% by mass, said emulsifying agent is contained in a proportion of 0.5 to 5.0% by mass, and said 1,3-propanediol is contained in a proportion of 10.0 to 20.0% by mass.

15. The cosmetic preparation according to claim 3 or 6, wherein said cosmetic preparation is a cleansing cosmetic preparation, the fatty acids constituting said 1,3-propanediol difatty acid ester are n-octanoic acid and n-decanoic acid, said 1,3-propanediol difatty acid ester is contained in a proportion of 5.0 to 30.0% by mass, said emulsifying agent is contained in a proportion of 0.5 to 5.0% by mass, and said 1,3-propanediol is contained in a proportion of 30.0 to 50.0% by mass.

16. The cosmetic preparation according to claim 13 or 14, further comprising a polyglycerin fatty acid ester.

17. The cosmetic preparation according to claim 16, wherein said polyglycerin fatty acid ester is an esterification product of one or two or more types of fatty acids selected from lauric acid, myristic acid, and oleic acid, with polyglycerin whose average degree of polymerization is 10.

18. The cosmetic preparation according to claim 16, wherein said polyglycerin fatty acid ester is an esterification product of lauric acid and polyglycerin whose average degree of polymerization is 10, and the saponification value of said esterification product is from 35 to 75.

19. The cosmetic preparation according to claim 18, wherein said polyglycerin fatty acid ester is contained in a proportion of 0.05 to 10.0% by mass.

20. The cosmetic preparation according to any one of claims 1 to 6 and claims 9 to 12, wherein said cosmetic preparation is a moisturizing cosmetic preparation.

21. The cosmetic preparation according to claim 9 or 10, wherein said cosmetic preparation is a moisturizing cosmetic preparation, the fatty acids constituting said 1,3-propanediol difatty acid ester are isostearic acids, said 1,3-propanediol difatty acid ester is contained in a proportion of 18.0 to 60.0% by mass, said emulsifying agent is contained in a proportion of 0.5 to 5.0% by mass, and said 1,3-propanediol is contained in a proportion of 10.0 to 20.0% by mass.

22. The cosmetic preparation according to claim 9 or 10, wherein said cosmetic preparation is a moisturizing cosmetic preparation, the fatty acids constituting said 1,3-propanediol difatty acid ester are isostearic acids, said 1,3-propanediol difatty acid ester is contained in a proportion of 5.0 to 18.0% by mass, said emulsifying agent is contained in a proportion of 0.05 to 5.0% by mass, and said 1,3-propanediol is contained in a proportion of 3.0 to 10.0% by mass.

23. The cosmetic preparation according to claim 9 or 10, wherein said cosmetic preparation is a moisturizing cosmetic preparation, the fatty acids constituting said 1,3-propanediol difatty acid ester are isostearic acids, said 1,3-propanediol difatty acid ester is contained in a proportion of 6.0 to 20.0% by mass, said emulsifying agent is contained in a proportion of 0.5 to 5.0% by mass, and said 1,3-propanediol is contained in a proportion of 10.0 to 20.0% by mass.

24. The cosmetic preparation according to claim 9 or 10, wherein said cosmetic preparation is a moisturizing cosmetic preparation, the fatty acids constituting said 1,3-propanediol difatty acid ester are isostearic acids, said 1,3-propanediol difatty acid ester is contained in a proportion of 2.0 to 30.0% by mass, said emulsifying agent is contained in a proportion of 0.05 to 5.0% by mass, and said 1,3-propanediol is contained in a proportion of 3.0 to 10.0% by mass.

25. The cosmetic preparation according to claim 3 or 6, wherein said cosmetic preparation is a moisturizing cosmetic preparation, the fatty acids constituting said 1,3-propanediol difatty acid ester are n-octanoic acid and n-decanoic acid, said 1,3-propanediol difatty acid ester is contained in a proportion of 3.0 to 10.0% by mass, said emulsifying agent is contained in a proportion of 0.05 to 5.0% by mass, and said 1,3-propanediol is contained in a proportion of 3.0 to 10.0% by mass.

26. The cosmetic preparation according to claim 3 or 6, wherein said cosmetic preparation is a moisturizing cosmetic preparation, the fatty acids constituting said 1,3-propanediol difatty acid ester are n-octanoic acid and n-decanoic acid, said 1,3-propanediol difatty acid ester is contained in a proportion of 20.0 to 50.0% by mass, said emulsifying agent is contained in a proportion of 3.0 to 10.0% by mass, and said 1,3-propanediol is contained in a proportion of 3.0 to 10.0% by mass.

27. The cosmetic preparation according to claim 26, further including 3.0 to 10.0% by mass of said 1,3-propanediol difatty acid ester the fatty acid of which is isostearic acid.

28. The cosmetic preparation according to claim 9 or 10, wherein said cosmetic preparation is a moisturizing cosmetic preparation, the fatty acids constituting said 1,3-propanediol difatty acid ester are isostearic acids, said 1,3-propanediol difatty acid ester is contained in a proportion of 8.0 to 50.0% by mass, said emulsifying agent is contained in a proportion of 0.05 to 5.0% by mass, and said 1,3-propanediol is contained in a proportion of 3.0 to 20.0% by mass.

29. The cosmetic preparation according to claim 9 or 10, wherein said cosmetic preparation is a moisturizing cosmetic preparation, the fatty acids constituting said 1,3-propanediol difatty acid ester are isostearic acids, said 1,3-propanediol difatty acid ester is contained in a proportion of 8.0 to 60.0% by mass, said emulsifying agent is contained in a proportion of 0.05 to 5.0% by mass, and said 1,3-propanediol is contained in a proportion of 8.0 to 25.0% by mass.

30. The cosmetic preparation according to any one of claims 1 to 29, wherein said emulsifying agent is hydrogenated lecithin.

31. The cosmetic preparation according to any one of claims 1 to 30, wherein plant-derived or microbial fermentation-derived raw materials account for 5 to 100% by mass in the entire raw materials of said cosmetic preparation.

32. A method for producing a cosmetic preparation according to any one of claims 1 to 25 and claims 27 to 31, wherein the method comprises making an oil-in-water (O/W) emulsified cosmetic preparation by adding said 1,3-propanediol difatty acid ester to a hydrophilic solution in which said emulsifying agent and said 1,3-propanediol have been previously dissolved or dispersed.

33. A composition for cosmetic preparations, which comprises a 1,3-propanediol difatty acid ester, wherein the fatty acids constituting said 1,3-propanediol difatty acid ester are one or two or more types of fatty acids selected from isostearic acid, n-octanoic acid, and n-decanoic acid, and said isostearic acid has a structure in which one or two methyl groups are branched from the principal chain of stearic acid.

34. The composition for cosmetic preparations according to claim 33, wherein the fatty acids constituting said 1,3-propanediol difatty acid ester are n-octanoic acid and n-decanoic acid.

35. The composition for cosmetic preparations according to claim 34, wherein the mass ratio of the n-octanoic acid and the n-decanoic acid which constitute said 1,3-propanediol difatty acid ester is from 95:5 to 5:95.

36. The composition for cosmetic preparations according to claim 33, wherein the fatty acids constituting said 1,3-propanediol difatty acid ester are isostearic acids.

37. The composition for cosmetic preparations according to claim 36, wherein said isostearic acid has the following properties: the viscosity with a shear of 10 Pa is from 99 to 130 mPa·s at 10°C, and the isostearic acid is in a liquid state at 5°C.

38. The composition for cosmetic preparations according to any one of claims 33 to 37, wherein said cosmetic preparation composition is for use as a cleansing cosmetic preparation.

39. The composition for cosmetic preparations according to any one of claims 33 to 37, wherein said cosmetic preparation composition is for use as a moisturizing cosmetic preparation.

40. The composition for cosmetic preparations according to claim 36 or 37, wherein said cosmetic preparation composition is for use as a substitute for a macadamia nut oil.

41. The composition for cosmetic preparations according to claim 34 or 35, wherein said cosmetic preparation composition is for use as a remover of an oil soluble colorant.

42. The composition for cosmetic preparations according to claim 34 or 35, wherein said cosmetic preparation composition is for use as a remover of an ink of an oil paint pen.

43. The composition for cosmetic preparations according to claim 42, wherein said cosmetic preparation composition is for use as a remover of an ink of an oil paint pen, and the mass ratio of the n-octanoic acid and the n-decanoic acid which constitute said 1,3-propanediol difatty acid ester is from 90:10 to 40:60.

44. The composition for cosmetic preparations according to claim 34 or 35, wherein said cosmetic preparation composition is for use as a substitute for a silicone oil.

45. The composition for cosmetic preparations according to any one of claims 33 to 44, wherein plant-derived or microbial fermentation-derived raw materials account for 5 to 100% by mass in the entire raw materials of said cosmetic preparation composition.

46. A cosmetic preparation comprising the composition for cosmetic preparations according to any one of claims 33 to 45.

47. A method for producing a cosmetic preparation, wherein the method comprises making an oil-in-water (O/W) emulsified cosmetic preparation by adding the composition for cosmetic preparations according to any one of claims 33 to 45, to a hydrophilic solution.

48. A cleanser for industrial use, which comprises 1,3-propanediol difatty acid ester, wherein the fatty acids constituting said 1,3-propanediol difatty acid ester are n-octanoic acid and n-decanoic acid.

49. The cleanser for industrial use according to claim 48, wherein the mass ratio of the n-octanoic acid and the n-decanoic acid which constitute said 1,3-propanediol difatty acid ester is from 95:5 to 5:95.

50. A cleanser for industrial use according to claim 48, wherein the mass ratio of the n-octanoic acid and the n-decanoic acid which constitute said 1,3-propanediol difatty acid ester is from 90:10 to 40:60.
